# EUROPEAN PATENT SPECIFICATION

(11) **EP 3 785 664 B1**
(45) Date of publication and mention of the grant of the patent: **13.03.2024**
(21) Application number: 19792243.8
(22) Date of filing: 12.04.2019
(51) Int. Cl.: A61C 17/34, A61C 17/38, A61C 9/00, A61C 17/028

(54) **MOUTHPIECE TEETH CLEANER**
ZAHNREINIGUNGSMUNDSTÜCK
APPAREIL DE NETTOYAGE DE DENTS DE TYPE EMBOUT BUCCAL

(30) Priority: 26.04.2018 CN 201810386151
(43) Date of publication of application: 03.03.2021
(73) Proprietor: Beijing Keeyoo Technologies Co., Ltd., Beijing 100085 (CN)
(72) Inventor: OUYANG, Congxing, Beijing 100033 (CN)
(74) Representative: FARAGO Patentanwälte GmbH
(86) International application number: PCT/CN2019/082554
(87) International publication number: WO 2019/205968

(56) References cited:
- WO-A1-2010/076702
- WO-A1-2017/035979
- WO-A1-2019/004720
- CN-A- 107 811 720
- CN-A- 108 903 982
- CN-U- 204 709 048
- CN-U- 204 971 671
- JP-A- 2017 094 020
- US-A- 4 164 940
- US-A1- 2017 265 638
- US-A1- 2018 098 832

## Description

### TECHNICAL FIELD

The invention belongs to the field of oral cavity cleaning daily necessities, and relates to a mouthpiece-type teeth cleaner.

### BACKGROUND ART

Bass method, also known as gingival sulcus cleaning or horizontal flutter, is an effective method to remove plaque near the gingival margin and in the gingival sulcus.

Bass method requires the following conditions: a soft-bristled toothbrush should be selected, the toothbrush form, with a long axis of the tooth, an angle of 45 degrees to point in the apical direction (upward for upper jaw tooth, downward for lower jaw tooth), the gum-tooth interface area is pressed, one part of the bristles enters the gingival sulcus, one part of the bristles are spread on the gingival margin and extend into the adjacent gap as far as possible, and the bristles are horizontally vibrated by soft pressure for 10 times in a short distance in the front-back direction *in situ.* The toothbrush moved only about 1 mm while vibrating. For the linguopalatine side of the anterior teeth, if the arch is narrow, the toothbrush can be upright. The bristle enters the gingival sulcus and adjacent space at an angle of about 45 degrees and makes a short tremor against the long axis of the tooth. For the maxillofacial region, the action of brushing teeth is to press the bristles against the maxillofacial region, so that the hair ends go deep into the point space and make vibrations in direction of the front and back teeth. However, since the gingival sulcus and gingival margin are both arched curves, for the existing toothbrush, or electric toothbrush, or tooth cleaning device, the natural arched curve of the gingival margin cannot be fitted due to a single movement direction, so that it is difficult to implement the Bass method at each part of the gingival margin/gingival sulcus.

Recently, a toothbrush (Blizzident) customized according to the user's teeth has been developed. Customization occurs because the user's teeth are first 3D scanned, modeled, and then the toothbrush is made targeted according to the tooth model. When using Blizzident, you can complete the entire brushing process by chewing more than ten toothbrushes up and down, left and right. Blizzident has more than about 400 soft bristles, and the soft bristles and gums form a 45 degree angle, and each corner can be cleaned as it is customized to the user's teeth. However, the toothbrush still has no personalized actuation capability, but relies solely on the overall relative movement between the upper and lower dentitions of the oral cavity and the bristles to clean the teeth. Therefore, the brushing action of the toothbrush cannot fit the natural arch curve of the gingival sulcus, and the requirement of the Bass method cannot be met.

US 4,164,940 discloses an apparatus for cleaning teeth and the gingival crevices and for massaging the gums, comprising a mouthpiece having an upper channel member adapted to fit over at least a part of the upper dentation and to snugly engage the upper gum and a lower channel member adapted to fit over at least a part of the lower dentation and to snugly engage the lower gum.

WO 2017/035979 discloses a dental cleaner consisting of a dental cover and a handle, wherein the dental cover comprises an upper teeth groove and a lower teeth groove, and open pores arranged at intervals are distributed within the upper and lower teeth grooves.

### SUMMARY OF THE INVENTION

Aiming at the defects in the prior art, the invention aims to solve the problem that the existing teeth-cleaning method and device cannot really implement the Bass method, and provide a mouthpiece-type teeth cleaner enabling a complete clean, efficient and convenient mouthpiece-type teeth-cleaning method for users.

The invention is set out in the appended set of claims.

Compared with the prior art, the mouthpiece-type teeth cleaner disclosed by the invention achieves the following technical effects:
1. According to the mouthpiece-type teeth cleaner disclosed by the invention, a user does not need to move the cleaning device everywhere in the oral cavity, and can effectively clean different positions of teeth at the same time only by performing occlusion and release actions, so that the resulting mouthpiece-type teeth-cleaning method is very convenient to use, and particularly can well clean tooth positions such as gingival sulcus, diastema cleft, lingual tooth surface, palatal tooth surface, distal molar distal and middle surface and the like.
2. According to the mouthpiece-type teeth cleaner disclosed by the invention, the space relation layout of the teeth of a user is obtained by taking a model of the teeth of the user, so that the mouthpiece matched with the dentition of the user is designed, through the establishment of the correlation between a cleaning system on the mouthpiece with the tooth model, positional scribing is carried out on different positions of the dentition of the user, and different brushing actions are adopted on different types of positional scribing, and then different positions of the teeth of the user (including gingiva grooves, tooth surfaces, tooth occlusal surfaces, interdental gaps and the like) can be cleaned individually, so that the requirement of the Bass method is met, and a tooth cleaner which is more comprehensive in cleaning, more efficient and convenient is provided for the user.
3. The teeth cleaner provides three cleaning operation modes for cleaning teeth, the three cleaning operation modes can be freely combined, different cleaning operation modes can be selected aiming at different positions of the teeth, and user experience is improved.

### BRIEF DESCRIPTION OF THE DRAWINGS

FIG. 1 is a schematic flow diagram illustrating a correlation establishing process by using a teeth-cleaner according to an embodiment of the present invention;
FIG. 2 is a schematic diagram of a process for establishing a digitized three-dimensional mouthpiece model according to an embodiment of the present invention;

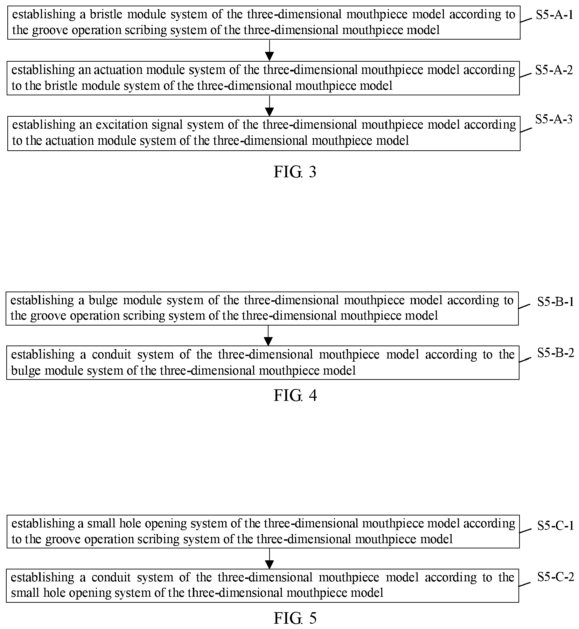

FIG. 3 is a schematic flow diagram illustrating the establishment of a cleaning operation system in operation mode I according to a first embodiment of the present invention;
FIG. 4 is a schematic flow diagram illustrating the establishment of a cleaning operation system in operation mode II according to a second embodiment of the present invention;
FIG. 5 is a schematic flow diagram illustrating the establishment of a cleaning operation system in operation mode III according to a third embodiment of the present invention;
FIG. 6 is a schematic diagram of a tooth model molded according to an embodiment of the present invention;
FIG. 7 is a block diagram of an adj acent tooth after being molded according to an embodiment of the present invention;

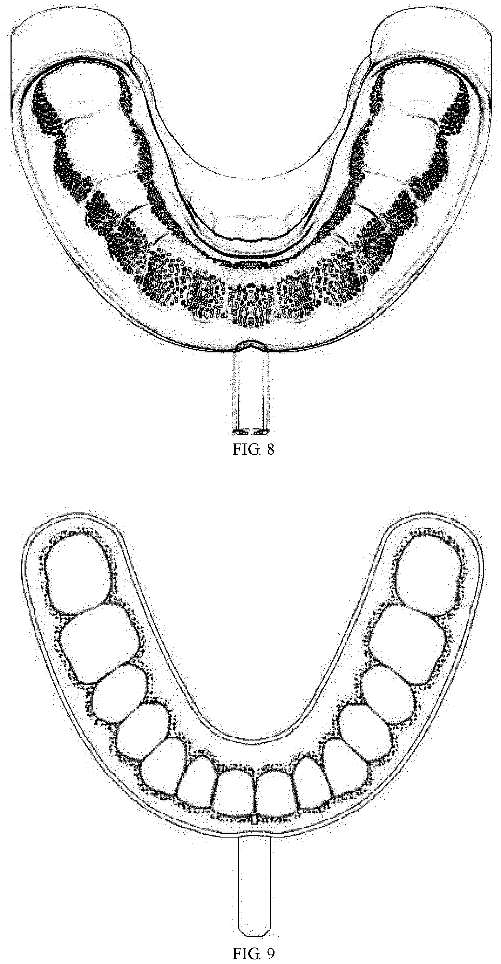

FIG. 8 is a schematic diagram showing a three-dimensional structure of a mouthpiece model according to an embodiment of the present invention;
FIG. 9 is a plan view of a phatnoma structure of a phatnoma model according to an embodiment of the present invention;
FIG. 10 is a schematic diagram showing a change state of a bulge in the second embodiment of the present invention;
FIG. 11 is a schematic diagram showing a change state of a bulge in the second embodiment of the present invention;
FIG. 12 is a schematic diagram showing a structure in which open holes are provided in the third embodiment of the present invention;
Fig. 13 is a structural principle diagram of a pneumatic driving unit according to the first embodiment of the present invention;
Fig. 14 is a structural principle diagram of the pneumatic driving unit according to to the first embodiment of the present invention;
Fig. 15 is structural principle diagram I of an actuation module according to the first embodiment of the present invention;
FIG. 16 is structural principle diagram II of the actuation module according to the first embodiment of the present invention;
Fig. 17 is structural principle diagram III of the actuation module according to the first embodiment of the present invention;
FIG. 18 is structural principle diagram IV of the actuation module according to the first embodiment of the present invention;
Fig. 19 is schematic diagram I showing a structure in which the actuation module of the first embodiment of the present invention is an artificial muscle;
Fig. 20 is schematic diagram II showing a structure in which the actuation module of the first embodiment of the present invention is an artificial muscle;
Fig. 21 is schematic diagram III showing a structure in which the actuation module of the first embodiment of the present invention is two artificial muscles;
Fig. 22 is schematic diagram IV showing a structure in which the actuation module of the first embodiment of the present invention is two artificial muscles;
Fig. 23 is schematic diagram V showing a structure in which the actuation module of the first embodiment of the present invention is four artificial muscles;
Fig. 24 is schematic diagram VI showing a structure in which the actuation module of the first embodiment of the present invention is four artificial muscles.

### DETAILED DESCRIPTION OF THE INVENTION

In order that those skilled in the art may better understand the technical aspects of the present invention, the present invention will be described in further detail with reference to the accompanying drawings and detailed description.

The invention aims at customizing the mouthpiece structure of a mouthpiece-type teeth cleaner for implementing the Bass method for a user according to the shape and layout of the whole mouth teeth of the user, thereby realizing a novel teeth-cleaning method based on the mouthpiece.

The mouthpiece-type teeth-cleaner enables a method disclosed by the embodiment of the invention, the method comprises:
generating one or more groups of alternating excitation signals by an excitation source;
generating one or more cleaning operations, with different cleaning actions applied to various parts of the teeth according to the excitation signals;
wherein, a correlation between the cleaning operation and the cleaning action and each part of applied tooth is established in advance, and the correlation enabling each position of the tooth of the user to be individually cleaned.

The present invention generates an excitation signal from an excitation source, which may be electric, pneumatic, or a combination thereof, in an alternating manner, *i.e*., a periodically alternating pattern, such as an intermittent pattern of alternating changes between active and inactive states. Upon activation of the activation signal, a cleaning operation, which may be a direct brushing action on the tooth surface, is controlled to be generated to effect cleaning of various positions of the tooth. The cleaning operation may be a direct applied brushing cleaning action against the tooth surface_{∘} Since the correlation between the cleaning operation and the cleaning action and each part of the teeth of the user is established in advance, the cleaning action can be different according to each part of the teeth of the user, such as: different parts such as a tooth surface, a tooth occlusal surface, a gingival sulcus, a diastema, a distal molar distal and middle surface and the like and different parts of teeth of different users have different sizes, areas, shapes and the like, and different cleaning actions are applied to carry out targeted cleaning. On the one hand, it is a fully automatic cleaning mode, on the other hand, it is also a personalized cleaning mode.

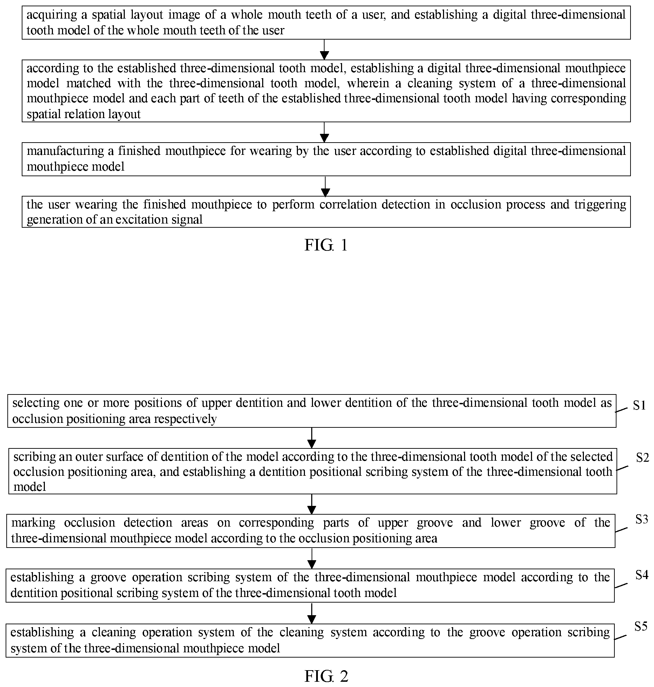

Referring to FIG. 1, in the embodiment of the present invention, the process for establishing the correlation includes:
Firstly, acquiring a spatial layout image of teeth of an oral cavity of a user, and establishing a digital three-dimensional tooth model of the teeth of the user.

The process for establishing the tooth model specifically comprises: carrying out image recognition on the full-mouth teeth or partial teeth of a user, wherein the recognition method comprises performing model taking or 3D scanning on the full-mouth dentitions (or partial dentitions) of the mouth of the user to obtain a spatial layout image of the teeth of the user, wherein the spatial layout image comprises the number of the teeth of the user, the shape and the size of a three-dimensional curved surface of the outer surface of each tooth, the distance between each adjacent tooth, the shape and the camber of a contact part between the tooth and the gingiva and the like, covering all spatial relation layouts of the medial (lingual, palatal) and lateral (labial, buccal), occlusal and incisal surfaces, and the distal and medial surfaces of the left and right distal molars of the user's teeth, thus obtaining a tooth model matched with the user's actual dentition.

Secondly, establishing a digital three-dimensional mouthpiece model matched with the three-dimensional tooth model according to the established three-dimensional tooth model, wherein a cleaning system is arranged on a groove of the three-dimensional mouthpiece model, the cleaning system and each part of teeth of the established three-dimensional tooth model have corresponding spatial relation layout, and cleaning of each part of teeth is realized by the cleaning system.

Thirdly, according to the established digital three-dimensional mouthpiece model, manufacturing a finished mouthpiece which is for wearing by the user.

Fourthly, the user wearing the finished mouthpiece, carrying out correlation detection in the occlusion process, triggering and generating the excitation signal after detecting the compliance, and then generating the cleaning action.

That is, the correlation includes: the correlation between the tooth model and the actual dentition of the user, the correlation between the upper and lower phatnomas of the mouthpiece model and the upper and lower dentitions of the tooth model, and the correlation between the cleaning system of the mouthpiece and each part of the groove of the mouthpiece model, and the correlation between the mouthpiece for wearing by the user and the actual dentition of the user are finally established.

In this embodiment, the cleaning operation is generated by the cleaning system and the cleaning operation mode is based on a combination of one or more of the following three modes:
Operation mode I:
a plurality of actuation modules connected with the excitation source are arranged in the mouthpiece, the actuation modules receiving the excitation signal and generating one or more groups of mechanical motions;
the mechanical motion driving a bristle module arranged on the mouthpiece to perform reciprocating brushing so as to clean relevant parts of teeth.

In operation mode I, the cleaning system consists of an excitation source, an actuation module and a bristle module, the actuation module is controlled to act after the excitation source is started, the actuation module drives the bristle module to act, the bristle module consists of bristle clusters, and each part of a tooth is brushed by the bristle clusters.

In operation mode I, the signal generated by the excitation source may be an alternating pneumatic, hydraulic signal or voltage signal.

Operation mode II:
a conduit is embedded in the mouthpiece, one end of the conduit is connected with the excitation source, a hollow bulge communicated with a channel of the conduit is arranged on a wall surface of the conduit, the excitation source generating alternating excitation signals to control the bulge to perform reciprocating straightening and bending actions so as to clean relevant parts of teeth.

In operation mode II, the cleaning system consists of a plurality of conduits embedded in a groove, a bulge and an excitation source. After the excitation source is started, gas or liquid is intermittently blown into the conduits, so that the bulges are straightened and bent in time, and the bulges are changed between the straightened state and the bent state to form a motion track to brush relevant parts of teeth.

In operation mode II, the signal generated by the excitation source may be an alternating pneumatic or hydraulic signal.

Operation mode III:
a conduit is embedded in the mouthpiece, one end of the conduit is connected with the excitation source, the other end of the conduit is provided with a small hole and corresponding to a relevant position of the tooth, and the excitation source spraying liquid, gas or vapor-liquid mixed mist to relevant position of the tooth through the conduit and the small hole so as to clean the relevant position of the tooth.

In operation mode III, the cleaning system consists of a plurality of conduits, small holes and an excitation source. After the excitation source is started, gas is blown in the conduit, liquid or mist is sprayed outwards through the small holes, and the sprayed gas, liquid or mist impacts the outer surface of the tooth to clean relevant parts of the tooth. It should be noted that the three modes of operation are primarily directed to the cleaning of the diastema.

In operation mode III, the signal generated by the excitation source is an alternating pneumatic or hydraulic signal.

With regard to the selection of the cleaning operation mode, one preferred embodiment is: cleaning of each tooth part is accomplished by a combination of three or any two of the cleaning operations described above.

For example: the diastema cleft, the gingival sulcus, the adjacent gingival part, the tooth surface, the tooth occlusal surface and other parts are brushed and cleaned by the projection reciprocating action in cleaning operation mode II; aiming at the position of the diastema, a mode of spraying cleaning liquid through a small hole of a conduit in a cleaning operation mode III is adopted at the same time.

For another example: the diastema cleft, the gingival sulcus, the adjacent gingival part, the tooth surface, the tooth occlusal surface and other parts are brushed and cleaned by a bristle module in cleaning operation mode I. Aiming at the position of the diastema, a mode of spraying cleaning liquid through a small hole of a conduit in cleaning operation mode III is adopted at the same time.

For another example: aiming at the position of the diastema cleft, the cleaning liquid is sprayed through the small holes of the conduits in cleaning operation mode III, and the operation is convenient. The bristle module in cleaning operation mode I is used for brushing and cleaning the positions of the diastema cleft, the gingival sulcus, the tooth surface, the tooth occlusal surface and the like, the cleaning is cleaner, the gingival position adjacent to the gingival sulcus is brushed and cleaned through the reciprocating motion of the bulges in cleaning operation mode II, and the discomfort of the gingival position is reduced.

Referring to FIG. 2, the process of establishing the digitized three-dimensional mouthpiece model matched with the three-dimensional tooth model specifically includes:
Step S 1: selecting one or more positions on upper dentition and lower dentition of the three-dimensional tooth model as occlusion positioning area.

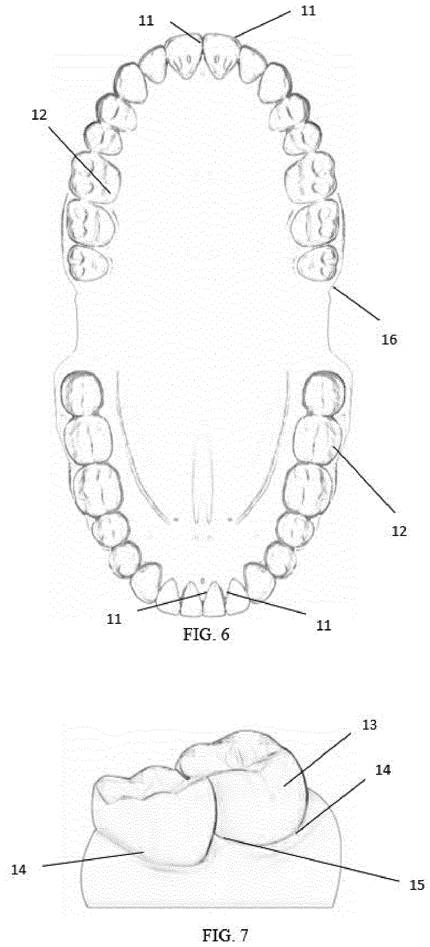

In particular, at least one (*i.e*., one or more) region of each of the upper and lower dentition of the digitized three-dimensional tooth model is selected as the occlusal positioning region, and the selected portion is a portion where the upper and lower dentition can touch, such as an apical portion. More preferably, two upper incisors (as shown by reference numeral 11 in the upper part of Fig. 6) and two lower incisors (as shown by reference numeral 11 in the lower part of Fig. 6) in the middle of the dentition of the user (a tooth section, and a labial tooth surface position area and a lingual-palatal tooth surface position area adjacent to the section) are selected as occlusion positioning area.

Step S2: scribing an outer surface of dentition of the model according to the three-dimensional tooth model of the selected occlusion positioning area, and establishing a dentition positional scribing system of the three-dimensional tooth model.

Specifically, step S2 specifically comprises:
Step S21: according to the three-dimensional tooth model of the selected occlusion positioning area, scribing other surface areas of the outer surface of the dentition of the model except the occlusion positioning area to form a plurality of mutually connected positional scribing; therein, the scribings are classified into several categories according to different cleaning requirements of various parts of teeth. For example, for other surface areas of the outer surface of the three-dimensional tooth model in the normal state except the occlusion positioning area, the scribings are classified into the following categories: gingival sulcus positional scribing, tooth surface positional scribing, diastema positional scribing, tooth occlusal surface positional scribing, tooth section positional scribing and distal molar distal and middle surface positional scribing. As another example, for other surface areas of the outer surface of the three-dimensional tooth model in a state where the metal bracket type dental appliance is worn than the occlusion positioning area, the scribings are classified into into the following categories: gingival sulcus positional scribing, tooth surface positional scribing, alveolar ridge and bracket diastema positional scribing, alveolar ridge side bracket sulcus positional scribing, apical side bracket sulcus positional scribing, bracket and apical diastema positional scribing, diastema positional scribing, alveolar ridge and bracket diastema positional scribing, bracket and apical diastema positional scribing, tooth occlusal surface positional scribing, tooth section positional scribing, and distal molar distimal positional scribing. Different classes of the positional scribings are preset in advance with different initial cleaning requirement parameters. The initial cleaning demand parameter may also include a selection of the three cleaning modes of operation described above, i.e., when the initial cleaning demand parameter is set, which cleaning mode is selected for which locations.

Specifically, the positional scribing is carried out on the basis of different brushing requirements of different parts. The gingival sulcus position refers to the area where the teeth are combined with the gums, and the gingival communication is usually an arch curve. The tooth surface part refers to the tooth surface area on the labial-buccal side and the lingual-palatal side of the teeth, the tooth occlusal surface part refers to the apical occlusal areas of the premolar teeth and the posterior molar teeth, and the tooth section position refers to the apical section area of the anterior teeth. The diastema region refers to the region formed by the gap between two adjacent teeth, and the distal molar mesiofacial region refers to the mesiofacial region of the left most molar and the rightmost molar near the occlusal space of the dentition. Those skilled in the art will appreciate that the scribings for their tooth locations may be the same or different for different user populations. Referring to FIGs. 6 and 7, reference numeral 11 is a dental section, reference numeral 12 is an occlusal surface, reference numeral 13 is a dental surface, reference numeral 14 is a gingival sulcus, reference numeral 15 is a diastema, and reference numeral 16 is a distal molar distal and middle surface.

Taking cleaning operation mode I as an example, the initial cleaning requirement parameters include: the static inclination angle between the bristle and each brushing part, the motion period of the bristle, the motion direction of the bristle, the motion amplitude and other parameters. The initial cleaning requirement parameters can also include the flexibility parameter of the bristle material, the elasticity parameter of the bristle material, the diameter parameter of the bristle and the like. The preset initial cleaning requirement parameters are partially different for different categories.

Specifically regarding scribing a gingival sulcus position, initial cleaning requirement parameters of the gingival sulcus position are set as follows: Binchotan antibacterial sharpening fine bristles are selected for the bristles, the diameter parameter of the bristles is 0.01 mm, and the static inclination angle between the bristles and the inner tooth surface of the gingival sulcus is 45 degrees; the action mode of the bristle is a reciprocating symmetrical periodic motion, and the action period of the bristle is 500 ms. The included angle between the action direction of the bristle and the tangential direction of the arch curve of the gingival sulcus is less than 15 degrees, and the action amplitude of the bristle is 1 mm in the gingival sulcus. Taking diastema positional scribing as an example, the initial cleaning requirement parameters are set as follows: a flexible spiral bristle is selected for the bristle, the diameter parameter of the bristle material is 0.02 mm, and the static inclination angle between the bristle and the diastema is 90 degrees; the bristle motion mode is a back-and-forth asymmetric periodic motion, and the bristle motion period is 1000 ms; the action directions of the bristles are as follows: brushing from the apical end of the alveolar ridge of the diastema to the apical direction, and returning from the apical direction to the apical direction of the alveolar ridge. The action time from the apical brush of the alveolar ridge to the apical direction is 200 ms, and the action time from the apical direction back to the apical direction of the alveolar ridge is 800 ms; the brush action amplitude is 60 degrees swing angle.

Taking operation mode II as an example, the initial cleaning requirement parameters include: intervals at which the bulges are arranged on the wall surface of the conduit tube, and three-dimensional shape data (including length, sectional shape and size of each section, bending direction) of each bulge in a non-excitation signal state are obtained.

For example, the initial cleaning requirement parameters set for cleaning the bulges of the gingival sulcus position are: the static inclination angle between the bulge axis and the inner tooth surface of the gingival sulcus is 45 degrees; the bulge motion mode is a reciprocating symmetrical periodic motion, and the bulge motion period is 800 ms; the bulge action direction is the tangential direction of the gingival sulcus arch arc curve, and the included angle is less than 15 degrees; the amplitude of the bulge motion is 2 mm run in the gingival sulcus.

The initial cleaning requirement parameters set for cleaning the bulges of the diastema position are as follows: the static inclination angle between the bulge axis and the diastema is 90 degrees; the bulge motion mode is a reciprocating asymmetric periodic motion, and the bulge motion period is 1000ms. The bulge action direction is as follows: brushing from the crest end of the alveolar ridge of the diastema to the direction of the cusp, and returning to the direction of the crest end of the alveolar ridge from the direction of the cusp. The action time from the apical brush of the alveolar ridge to the apical direction is 200ms, and the action time from the apical direction back to the apical direction of the alveolar ridge is 800ms. The amplitude of the bump motion is 60 degrees swing angle.

Taking operation mode III as an example, the initial cleaning requirement parameter includes: the opening position of the small hole of the conduit (for example: aligning the position, which is 1.5 mm away from the crest end of the alveolar ridge, of the diastema, the size of the aperture (for example: 0.5 mm), and the jet speed of the fluid (for example: 1 m/s).

Step S22: respectively establishing the dentition spatial layout data and the cleaning requirement data of the dentition positional scribings according to the image information of the dentition positional scribings and the initial cleaning requirement parameters of the dentition positional scribings after the dentition positional scribings and dividing are completed, wherein all the positional scribings and data attached thereto form the dentition positional scribing system of the tooth model. The initial cleaning requirement parameters give initial values of default states of scribing cleaning requirement data of various parts, and most parameters in the cleaning requirement data can be consistent with the values in the initial cleaning requirement parameters.

Specifically, the dentition positional scribing is oriented toward tooth to clean, and its cleaning demand data includes its cleaning operation mode (*i*.*e*., one or more of the three afore mentioned cleaning operation modes) selection and initial cleaning demand parameters, and its spatial layout data is based on captured dentition image information.

Taking cleaning operation mode I as an example, the types of dentition scribing and the setting of cleaning requirement data are described in detail:
Firstly, for the gingival sulcus positional scribing, the gingival sulcus position corresponds to a corresponding gingival sulcus of a user tooth, and the set cleaning requirement data are as follows: Binchotan antibacterial sharpening fine bristles are selected for the bristles, the diameter parameter of the bristles is 0.01 mm, and the static inclination angle between the bristles and the inner tooth surface of the gingival sulcus is 45 degrees; the action mode of the bristle is a reciprocating symmetrical periodic motion, and the action period of the bristle is 500 ms. The included angle between the action direction of the bristle and the tangential direction of the arch curve of the gingival sulcus is less than 15 degrees, and the action amplitude of the bristle is 1mm in the gingival sulcus.

Secondly, for the tooth surface positional scribing, which has a corresponding tooth surface corresponding to the tooth surface positional scribing except for the occlusion positioning positional area, the cleaning requirement data set by the invention are as follows: brushing from root to tip. When the bristle is operated from the tooth root to the tooth tip direction, the speed is relatively high, and the strength is large. On the contrary, when the bristle returns from the tip to the root direction, the speed is relatively slow, and the strength is small. The stroke of the brushing action is 3-7 mm. In order to realize the brushing operation with asymmetric back-and-forth force, the alternating excitation signal needs to be designed in a waveform mode, so that the rising edge of the signal is steeper, and the falling edge is milder.

Thirdly, for the diastema positional scribing, the diastema position is formed by enclosing a plurality of curved surfaces of adjacent teeth of a user. The cleaning requirement data set by the invention are as follows: a flexible spiral bristle is selected for the bristle, the diameter parameter of the bristle material is 0.02 mm, and the static inclination angle between the bristle and the diastema is 90 degrees; the bristle motion mode is a back-and-forth asymmetric periodic motion, and the bristle motion period is 1000 ms; the action directions of the bristles are as follows: brushing from the apical end of the alveolar ridge of the diastema to the apical direction, and returning from the apical direction to the apical direction of the alveolar ridge. The action time from the apical brush of the alveolar ridge to the apical direction is 200ms, and the action time from the apical direction back to the apical direction of the alveolar ridge is 800ms; the brush action amplitude is a 60-degree swing angle. In order to realize the brushing operation with asymmetric back-and-forth force, the intermittent excitation signal needs to be designed in a waveform mode, so that the rising edge of the signal is steeper, and the falling edge is milder.

The gap between the diastema positional scribing and the slot body operation scribing is relatively large, about 5-9 mm. Thus, the length of bristles required at this location is relatively long.

Fourthly, for the tooth occlusal surface positional scribing, which corresponds to the occlusal surface of each molar of the user, the occlusal surface positional scribings all have a corresponding tooth surface corresponding thereto. The cleaning requirement data set by the invention are as follows: brushing back and forth along the horizontal direction of the plane of the occlusal surface, wherein the stroke of the brushing action is 3-7 mm.

Fifthly, for tooth section positional scribing, which corresponds to the section of each front tooth of the user, on the three-dimensional model of the dentition of the user, the section positional scribings have a corresponding tooth section corresponding to the corresponding tooth section. The cleaning requirement data set by the invention are as follows: horizontal brushing brush. Of course, no cleaning operation can be performed for this location, *i.e.* no bristle module and corresponding actuation module are provided on the scribing of the corresponding slot operation location on the phatnoma.

Sixth, for the tooth distal molar distal and middle surface positional scribing corresponding to the left most molar and the rightmost molar of the tooth near the distal and middle area of the occlusal space, the distal molar distal and middle surface position has a corresponding molar distal and middle surface. The cleaning requirement data set by the invention are as follows: brushing from root to tip. When the bristle is operated from the tooth root to the tooth tip direction, the speed is relatively high, and the strength is large. On the contrary, when the bristle returns from the tip to the root direction, the speed is relatively slow, and the strength is small. The stroke of the brushing action is 3-7 mm. In order to realize the brushing operation with asymmetric back-and-forth force, the alternating excitation signal needs to be designed in a waveform mode, so that the rising edge of the signal is steeper, and the falling edge is milder.

Step S3: marking occlusion detection areas on corresponding parts of upper groove and lower groove of the three-dimensional mouthpiece model according to the occlusion positioning area.

Specifically, when the tangent planes of certain two front teeth of the digitized three-dimensional tooth model and the adjacent areas thereof are selected as occlusion positioning area, correspondingly, the end parts, corresponding to the occlusion positioning area, of the central part of the surface of the groove of the mouthpiece model are designed as occlusion detection areas, and the parts outside the occlusion detection areas are cleaning operation areas. That is, the cut surface of the upper front tooth corresponds to the bottom end surface of the middle trough body of the upper trough body of the shell, and the cut surface of the lower front tooth corresponds to the top end surface of the middle trough body of the lower trough body of the shell. The bottom end face of the surface of the middle section groove of the upper groove of the mouthpiece and the top end face of the surface of the middle section groove of the lower groove of the mouthpiece are respectively used as two occlusion detection areas.

Step S4: establishing a groove operation scribing system of the digital three-dimensional mouthpiece model according to the dentition positional scribing system of the three-dimensional tooth model.

Wherein, step S4 specifically comprises:
Step S41: after finishing the dentition positional scribings of the three-dimensional tooth model and marking the occlusion detection area of the three-dimensional mouthpiece model, according to the corresponding relation with the upper dentition and the lower dentition of the tooth model, and according to the dentition spatial layout data of each dentition positional scribing and the cleaning requirement data corresponding to the selected cleaning operation mode, on the three-dimensional mouthpiece model, marking a groove operation scribing at a corresponding position of a surface area of the surface of the lower groove except for the occlusion detection area, and dividing the groove operation scribing into the following categories according to different corresponding dentition positional scribings: gingival sulcus operation scribing, tooth surface operation scribing, diastema operation scribing, tooth occlusal surface operation scribing, tooth section operation scribing and distal molar distal and middle surface operation scribing.

Step S42: after the marking of the groove operation scribing is finished, establishing spatial layout data of each groove operation scribing, wherein when the spatial layout data enables the occlusion detection area to abut against the occlusion positioning area, a gap existing between each groove operation scribing and each corresponding dentition positional scribing; the spatial layout data of the groove operation scribing comprising gap data between each groove operation scribing and the corresponding dentition positional scribing, and the gap data can be the same or different at different positions.

Step S43: establishing operation requirement data of each groove operation scribing according to the spatial layout data of each groove operation scribing, the spatial layout data of each corresponding dentition positional scribing and the cleaning requirement data corresponding to the selected cleaning operation mode, wherein all the groove operation scribing and the data attached to all the groove operation scribing form a groove operation scribing system of the mouthpiece model.

In addition, in the step, a space for installing a pressure sensor is reserved in the groove, and the pressure sensor is used for detecting whether the occlusion positioning area and the occlusion detection area are abutted and whether the occlusion force reaches the standard.

Since the outer surface of the tooth model is divided into a plurality of scribings, each of which has a specific shape, correspondingly, a phatnoma-operated scribing having a spatial relation layout consistent with that of the dentition positional scribings on the tooth model is provided on the mouthpiece model. The spatial relation layout of the dentition positional scribing array and the groove operation scribing array is composed of an "upper dentition positional scribing array - upper groove operation scribing array marking spatial relation layout", and a "lower dentition positional scribing array - lower groove operation scribing array marking spatial relation layout".

The "upper dentition positional scribing array - upper groove operation scribing array marking space relation layout" is: when the occlusion positioning area of the upper dentition surface of the user abuts against the "occlusion detection area" of the upper dentition surface of the mouthpiece, the occlusion detection area in the upper groove of the user abuts against the occlusion positioning area of the upper dentition of the user, and all other groove operation scribings in the upper groove of the user (including a gingival sulcus operation scribing, a tooth surface operation scribing, a tooth occlusion surface operation scribing, a tooth section operation scribing, an diastema operation scribing and a distal molar distal and middle operation scribing) have certain spatial correlation with the scribings of other dentition positions on the upper dentition of the user, and certain gaps (such as 6 mm ± 3 mm) are reserved between the corresponding scribings with correlation.

The "lower dentition positional scribing array - lower groove operation scribing array marking spatial relation layout" is consistent with the spatial relation layout of the upper dentition and the upper groove, and will not be described in detail herein. That is, since the bite detection region is located at a position where the bite detection region is required to be in direct contact with the bite, no cleaning operation is applied, while for the remaining portion (the portion other than the bite location region) cleaning operation is required, and a certain gap is left between the dentition positional scribing and the groove operation scribing. Because the extension part of the bristle or the projection extending out of the surface of the groove has f length, the gap provides a spreading space for the reciprocating brushing action of the bristle or the reciprocating stretching and bending action of the projection, and the size of the gap is different for different dentition positional scribings. For example, in gingival sulcus and tooth surface, the gap is relatively small; in the diastema, the space is relatively large.

The operation requirement data of the groove operation scribing receives the cleaning requirement data of the corresponding dentition positional scribings, which is not a value range, but a specific numerical value of the operation requirement data of each groove operation scribing established according to the spatial layout data of each groove operation scribing and the cleaning requirement data of each corresponding dentition positional scribings.

Step S5: establishing a cleaning operation system of the cleaning system according to the groove operation scribing system of the three-dimensional mouthpiece model.

In the following, the process of establishing the cleaning operation system of the three-dimensional mouthpiece model according to the present invention will be described by three specific examples.

### Embodiment 1

In this embodiment, tooth cleaning is achieved by means of cleaning operation mode I which is as follows: a plurality of actuation modules connected with the excitation sources are arranged in the mouthpiece, and the actuation modules receive the excitation signals and generate one or more groups of mechanical motions; the mechanical motion drives the bristle module arranged on the mouthpiece to perform reciprocating brushing so as to clean relevant parts of teeth.

The occlusion positioning area and the occlusion detection area are calibrated in advance, the dentition positional scribing system and the groove operation scribing system are established, and the cleaning operation system of the cleaning system needs to be established according to the groove operation scribing system.

In the embodiment, the process for establishing the cleaning operation system specifically comprises:
Step S5-A-1: establishing a bristle module system of the three-dimensional mouthpiece model according to the groove operation scribing system of the three-dimensional mouthpiece model.

In step S5-A-1, after a groove operation scribing system of the three-dimensional mouthpiece model is established, the bristle modules on each groove operation scribing are arranged, and bristle spatial layout data and brushing requirement data of each bristle module are established according to groove spatial layout data and operation requirement data of each groove operation scribing and dentition spatial layout data of each corresponding dentition positional scribing, and thus all the bristle modules and data attached thereto form a bristle module system of the mouthpiece model.

The upper groove and the lower groove of the mouthpiece are designed, so that the layout of the bristle module arrays of the upper groove and the lower groove respectively has a corresponding relation with each scribing except the occlusion detection operation scribing in the upper groove and the lower groove operation scribing arrays.

The space relation layout of the "groove operation scribing array - groove brushing module array" is: when the occlusion positioning area of the surface of the dentition of the user abuts against the occlusion detection area of the surface of the dentition groove, the array of the bristle modules of the dentition groove of the user has "correlation" with scribing of various parts of the dentition of the user.

The "correlation" is represented by the fact that each bristle module on the user's phatnoma has a corresponding relationship in spatial layout with each positional scribing of the user's dentition, and the corresponding relationship here can be simply understood as specifying different actions in advance for different positions of teeth, such as tooth surface, gingival sulcus and the like, so that customized brushing can be performed according to the positions with different motions preset. On the parameters of the bristle material, the bristle size (length and thickness), the bristle implantation density, the brushing action direction, the brushing action amplitude and the like, each bristle module corresponding to the spatial layout of each positional scribing of the user dentition can be different according to different cleaning requirements of each positional scribing of the user dentition.

The spatial layout data of the bristle module in the operation scribing of the groove comprises position data of each bristle in the scribing of the groove on the surface of the scribing of the groove, and three-dimensional shape data (including length, sectional shapes and sizes of each section and the extending direction of each bristle) of each bristle in the non-excitation signal state.

Step S5-A-2: establishing an actuation module system of the three-dimensional mouthpiece model according to the bristle module system of the three-dimensional mouthpiece model.

In step S5-A-2, after the bristle module system of the three-dimensional mouthpiece model is established, each corresponding actuation module drives each bristle module to act is arranged in phatnoma of the mouthpiece model, and the actuation spatial layout data and the actuation requirement data of each corresponding actuation module are established according to the bristle spatial layout data and the brushing requirement data of each bristle module, and all the actuation modules and data attached thereto form the actuation module system of the mouthpiece model.

Since the bristle module is driven to move only by the action of the actuation module, after the space position layout relation of the bristle module is set, corresponding actuation modules are designed for the bristle module in the upper groove and the lower groove of the mouthpiece position. The action direction and the action amplitude of the actuation module make corresponding customized design according to parameters such as the brushing action direction and the brushing action amplitude of the corresponding bristle module.

In the embodiment, the mouthpiece is composed of an outer coating layer made of a soft elastic material and an inner framework which is used for supporting the mouthpiece and is made of a hard material, the bristle module comprises a tray and bristles positioned on the tray, the bristles penetrate out of the surface of the outer coating layer, and the actuation module is positioned inside the mouthpiece and closed by the outer coating layer.

In the embodiment, the actuation module comprises a driving unit and a transmission unit, wherein the driving unit is connected with an excitation source and is used for receiving an excitation signal generated by the excitation source and driving the transmission unit to act, and the transmission unit is connected with a bristle module so as to drive the bristle module to act in a set manner.

The driving unit may be a pneumatic driving unit or a voltage driving unit.

The function of the pneumatic driving unit is as follows: converting the air pressure change conducted by the excitation source through the main conduit and the branch conduit into the telescopic action of the air pressure driving unit, wherein the excitation source is an air pump and particularly can be a piston pump arranged in the handle, and when the pumping action is executed, the most atmospheric pressure of the excitation source is matched with the air pressure required by the air pressure actuation module to reach the rated action amplitude, such as: the most atmospheric pressure may be 1.5 standard atmospheres.

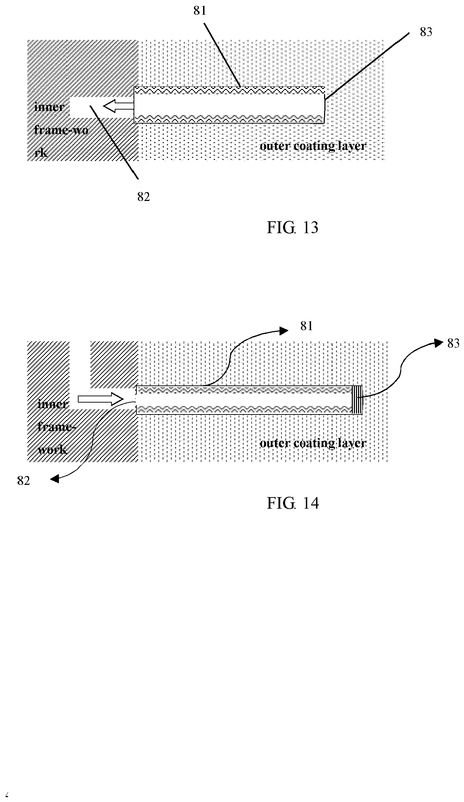

Referring to FIGs. 13 and 14, the pneumatic driving unit in this embodiment is a semi-hollow tube 81 in the shape of a cylinder, and the wall surface of the semi-hollow tube has elastic telescopic activity in the direction of the long axis of the cylinder. The open end of the semi-hollow tube is fixed to the inner framework of the shell portion and is communicated with a branch conduit 82 in the shell, and the closed end of the semi-hollow tube has no fixed relation with the framework of the shell portion and has mobility. One side of the closed end is provided with an end cap 83, which is connected to the transmission unit.

When the air pump in the handle performs a pumping action, the conduit and the branch conduit conduct high air pressure to the semi-hollow tube 81, so that the air pressure in the semi-hollow tube 81 increases, thereby causing the length of the semi-hollow tube 81 to increase, that is, the distance between the closed end and the open end to increase, as shown in FIG. 14. When the pumping action of the air pump in the handle is finished to withdraw, the air pressure in the branch conduit is reduced, so that the air pressure in the semi-hollow tube 81 is reduced, thereby causing the length of the semi-hollow tube to be shortened, that is, the distance between the closed end and the open end to be reduced, as shown in FIG. 13. When the air pump reciprocates to perform pumping action, the semi-hollow tube 81 of the air pressure driving unit reciprocates to perform stretching and shortening action, that is, to move left and right in the figure, so as to drive the end cover 83 on one side of the closed end of the semi-hollow tube to reciprocate to perform stretching movement.

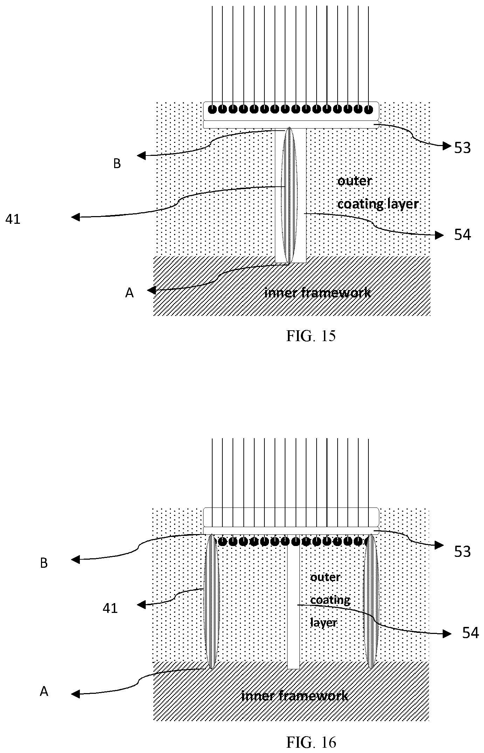

The function of the voltage driving unit is as follows: converting the voltage change conducted by the excitation source through the electric lead into the telescopic action of the voltage driving unit, wherein the excitation source is an electric signal. Referring to Figs. 15-18 in combination, in one embodiment of the present invention, the voltage driving unit is a strip-shaped artificial muscle 41 composed of an electrochemical polymer and having telescopic activity, wherein both ends of the strip-shaped artificial muscle 41 are respectively used as two electrodes (A, B) with different polarities, the electrodes are connected to a power source inside a handle through a lead, and the strip-shaped artificial muscle 41 is connected to or closely adjacent to the transmission unit.

When the power supply applies a voltage to the electrodes across the artificial muscle 41, the artificial muscle contracts. When the voltage on the electrodes (A, B) at both ends of the artificial muscle 41 is released, the artificial muscle is stretched and restored to the original shape. When a power source reciprocally applies a voltage to the electrodes at both ends of the artificial muscle 41 and releases the voltage, the artificial muscle 41 is driven to reciprocate in a contracting and elongating stretching motion. When the artificial muscle is driven to act, the maximum voltage of the artificial muscle is matched with the voltage required by the voltage driving unit to reach the rated action amplitude. Such as: the maximum voltage may be 24 volts. The turn-on and turn-off of the voltage driving unit can be controlled by the pulse of the single chip microcomputer.

The function of the transmission unit is as follows: converting the telescopic action of the driving unit into the swinging brushing action of the bristle module.

In one embodiment of the invention, the transmission unit is a 7-shaped connecting rod or a T-shaped supporting rod, wherein the 7-shaped connecting rod comprises a first cross rod and a first vertical handle which are integrally formed, and the T-shaped supporting rod comprises a second cross rod and a second vertical handle which are integrally formed.

The first cross rod of the 7-shaped connecting rod is flat, the first cross rod is fixedly connected with the tray of the bristle module or embedded into the tray, and the first vertical handle is connected with the air pressure driving unit or the voltage driving unit; the second cross rod of the T-shaped supporting rod is flat, the second cross rod is fixedly connected with the tray of the bristle module or embedded into the tray, and the second vertical handle is fixed on the mouthpiece.

Referring to FIGs. 15 and 16, in which FIG. 16 is a front elevation view and FIG. 15 is a side elevation view, a second vertical handle 54 of the T-shaped support bar is fixedly connected to the inner framework of the mouthpiece, two artificial muscles 41 are respectively arranged at two ends of the same inner side of a second cross bar 53 of the T-shaped support bar, and an attachment point is arranged at two ends of each artificial muscle 41. One of the attachment points (end A) is fixed to the inner frame, and the other attachment point is a free end (end B) and is connected to the second cross bar 53.

Therefore, according to the difference between the two pneumatic driving units and the transmission units, the actuation module has four construction modes: pneumatic driving + 7-shaped connecting rod transmission, pneumatic driving + T-shaped supporting rod transmission, voltage driving + 7-shaped connecting rod transmission and voltage driving + T-shaped supporting rod transmission.

These four solutions are further described below.

### Solution I: pneumatic driving + 7-shaped connecting rod transmission.

In Solution I, the actuation module is composed of a pneumatic driving unit and a 7-shaped connecting rod. When the closed end of the semi-hollow tube 81 of the actuation module reciprocates to perform telescopic movement, the first vertical handle of the 7-shaped connecting rod is pushed and pulled to reciprocate, so that the first transverse handle of the 7-shaped connecting rod is driven to reciprocate and swing, and the tray of the bristle module is driven to reciprocate and swing, so that bristle clusters implanted on the tray perform brushing actions back and forth according to the customized direction and amplitude.

### Solution II: pneumatic driving + T-shaped support rod transmission

In Solution II, the actuation module consists of a pneumatic actuation unit and a T-shaped supporting rod. When the closed end of the semi-hollow tube 81 of the actuation module reciprocates for telescopic movement, the tray-shaped second transverse handle of the T-shaped supporting rod is pushed and pulled to reciprocate and swing, so that the tray of the bristle module is driven to reciprocate and swing, and bristle clusters implanted on the tray perform brushing actions back and forth according to the customized direction and amplitude.

### Solution III: voltage driven + 7-shaped connecting rod transmission

In Solution III, the actuation module is composed of a voltage driving unit and a 7-shaped connecting rod. When the bar-shaped artificial muscle of the actuation module reciprocates to perform telescopic motion, the first vertical handle of the 7-shaped connecting rod is pushed and pulled to perform reciprocating motion, so that the first transverse handle of the 7-shaped connecting rod is driven to perform reciprocating swing, and the tray of the bristle module is driven to perform reciprocating swing, so that bristle clusters implanted on the tray perform brushing actions back and forth according to the customized direction and amplitude.

### Solution IV: voltage drive + T-shaped support rod transmission

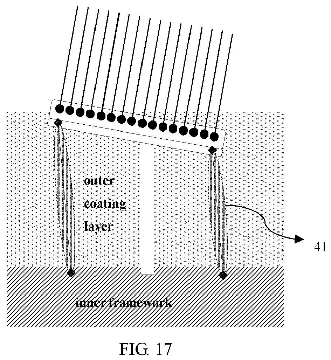

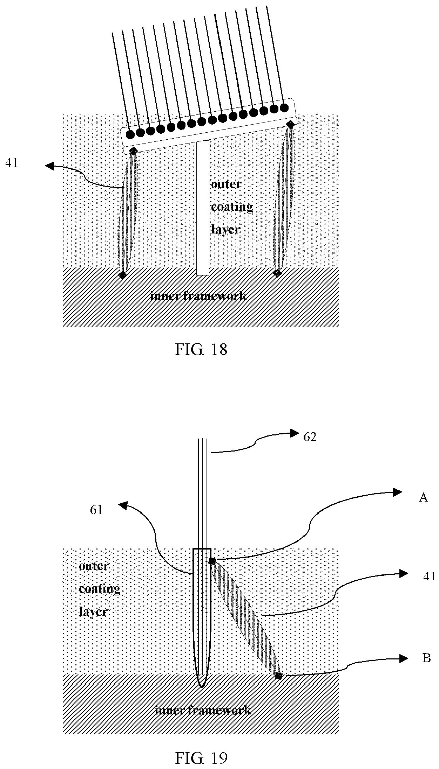

Referring to FIGs. 15-18, in Solution IV, the actuation module is composed of a voltage driving unit and a T-shaped support bar. When the bar-shaped artificial muscle 41 of the actuation module reciprocates to perform telescopic motion, the tray-shaped second transverse handle 53 of the T-shaped supporting rod is pushed and pulled to reciprocate and swing, and then the tray of the bristle module is driven to reciprocate and swing to form a changing state as shown in figures 17 and 18, so that bristle clusters implanted on the tray perform brushing actions back and forth according to the customized direction and amplitude.

There may be hundreds (e.g., 200-800) of bristle modules on the upper and lower groove surfaces of the mouthpiece portion of a dentifrice, respectively. A plurality of bristles (e.g., 10-100 bristles) are implanted into each bristle module to form a bristle cluster, alternatively, one bristle module corresponds to one actuation module, or a plurality of bristle modules with the same or substantially the same action amplitude and direction share the same actuation module, so that the arrangement number of the actuation modules can be reduced.

In another embodiment of the invention, the actuation module is an artificial muscle, the bristle module consists of a flocking tray and one or a cluster of bristles fixed by the flocking tray, the actuation source is a voltage signal, and the actuation module can directly pull the bristle module by one or more artificial muscles without the transmission unit in the solution I to Solution IV The flocking tray is in a vertical tube shape, the bottom of the tray is fixed on the inner framework, a single bristle or bristle cluster is fixed on the top of the tray, and at least one artificial muscle is arranged. One end of the artificial muscle is connected with the flocking tray, the other end of the artificial muscle is fixed on the inner framework, two ends of the artificial muscle are respectively used as two different electrode points, and the flocking tray is surrounded when the artificial muscle has a plurality of bristles. The excitation source controls the artificial muscle to contract or relax by applying voltage to the electrode points of the artificial muscle so as to drive the tray to act.

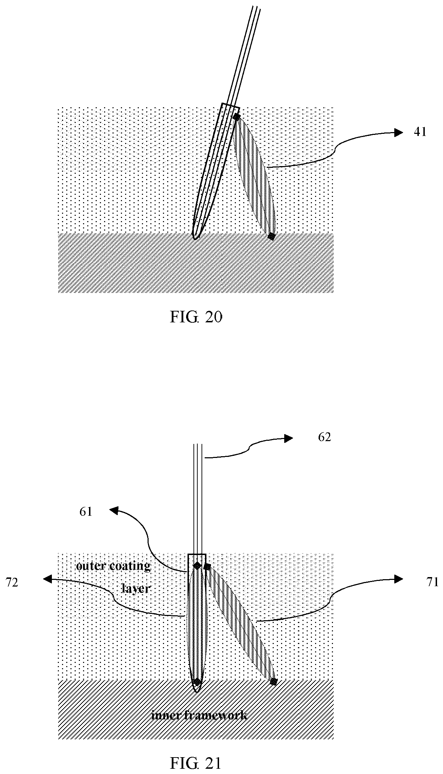

Referring specifically to FIGs. 19 and 20, in the present embodiment, the artificial muscle 41 is one which controls the action of the tubular flocking tray 61 to which a set of voltage excitation signals are applied. This causes the flocking tray 61 to perform a reciprocating tilting action in a specific direction and a specific amplitude, thereby driving the bristles 62 to perform a brushing operation. FIG. 19 is a schematic diagram showing the artificial muscle 41 in a relaxed state, without applying a voltage signal, when the tray 61 is erected, and FIG. 20 is a schematic diagram showing the artificial muscle 41 after applying a voltage signal to the artificial muscle, when the artificial muscle 41 is contracted and the flocking tray 61 is pulled to incline, so that the bristles 62 are inclined, thereby realizing a brushing action.

Because of the structural particularity of the gingival sulcus position, better cleaning effect can be brought by brushing along different directions of the gingival sulcus curve respectively. In a preferred embodiment of the invention, aiming at the gingival sulcus position, a bristle module is set to be controlled to brush by at least two artificial muscles, a plurality of artificial muscles are arranged around the flocking tray of the bristle module, the plurality of artificial muscles are set to be driven by a plurality of sets of excitation signals with phase differences, and the plurality of sets of excitation signals are set, by cooperation of the excitation signals with different voltage phase differences, such that when part of the artificial muscles contract, the rest of the artificial muscles are stretched, and the bristle module is driven to move towards the contraction direction of the artificial muscles. The bristle module is therefore driven to have a plurality of different brushing angles to the gingival sulcus position.

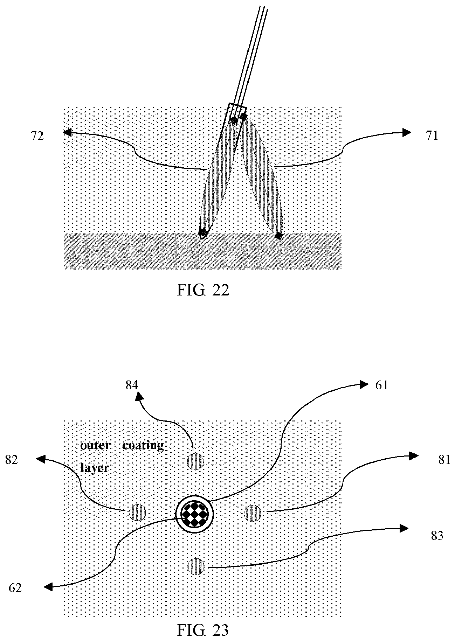

FIGs. 21 and 22 show another schematic structural diagram of an actuator module according to the invention. In this embodiment, two artificial muscles (71 and 72, respectively) are provided at ninety degrees, the actual mounting direction of the two artificial muscles is arranged such that one extends along a tangential direction of the curve of the gingival sulcus and another extends at a ninety degree arrangement along the normal direction of the curve of the gingival sulcus. One end of each artificial muscle (71, 72) is fixedly connected with the upper part of the flocking tray 61, the other end of each artificial muscle is fixed on the inner framework to form two electrode points, and each artificial muscle is respectively connected with a set of voltage excitation signals of different phases. That is, two sets of voltage excitation signals are summed. The two sets of voltage excitation signals enable the contraction actions of the two artificial muscles to be matched with each other through the matching of the voltage signal phases, namely when one artificial muscle is electrified, the other artificial muscle is not electrified. At the moment, the electrified artificial muscle contracts, the electrified artificial muscle relaxes, and when the two artificial muscles are in electrified and non-electrified states with each other, the bristles 62 are alternately brushed in the tangential direction of the gingival sulcus curve and the normal direction of the gingival sulcus curve.

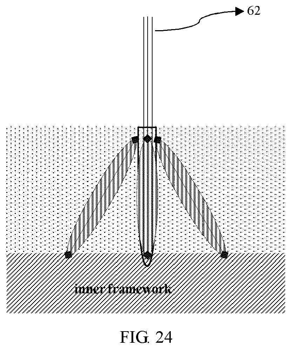

Although the use of two artificial muscles arranged at ninety degrees enables the bristles to alternate in the tangential direction of the gingival sulcus curve and the normal direction of the teeth, the two artificial muscles have a factor of instability in the direction of motion, as shown in FIGs. 23 and 24, wherein FIG. 23 is the top view thereof. In a preferred embodiment, four artificial muscles (91, 92, 93 and 94) are used. The four artificial muscles are arranged with 90 degrees around the flocking tray. In the present embodiment, the artificial muscle 91 and the artificial muscle 92 are arranged in the tangential direction of the gingival sulcus, and the artificial muscle 93 and the artificial muscle 94 are arranged in the normal direction of the gingival sulcus. Each artificial muscle is coupled to a set of voltage excitation signals of different phases, *i.e.* four sets of voltage excitation signals are summed. The four groups of voltage excitation signals are matched with each other through the phases of the voltage signals, so that the stretching and contracting actions of the four artificial muscles are matched with each other in various ways, the flocking tray 61 is driven to make reciprocating tilting actions according to various specific directions and specific amplitudes, and the brushing operation is executed. For example, when the artificial muscle 91 is electrified and the other three artificial muscles are not electrified, the flocking tray 61 moves in the direction of the artificial muscle 91. When the artificial muscle 94 is electrified and the other three artificial muscles are not electrified, the flocking tray moves in the direction of the artificial muscle 94, and the adjacent two artificial muscles are arranged at an included angle of ninety degrees. The bristle module 62 is thus alternately made to brush in the tangential direction of the gingival sulcus curve and the normal direction of the gingival sulcus curve.

The specific layout and operation modes of the bristle module and the actuation module are exemplified as follows:
First brushing action: the brushing action is performed by the bristle module facing the gingival sulcus positional scribing.

The bristle module facing the gingival sulcus positional scribing comprises a gingival sulcus positional scribing of each tooth on the jaw side of the upper dentition, a gingival sulcus positional scribing of each tooth on the cheek side of the upper dentition, a gingival sulcus positional scribing of each tooth on the tongue side of the lower dentition and a gingival sulcus positional scribing of each tooth on the cheek side of the lower dentition corresponding to the gingival sulcus positional scribing of each tooth of a user. Soft and elastic sharpening fine bristles are adopted for the bristles on the gingival sulcus bristle module. Due to the design, the bristles on the gingival sulcus bristle module can easily extend into the gingival sulcus to remove stains such as plaque and the like, and the gums are not damaged. Under the driving of the actuation module, the bristle module of each gingival sulcus position performs a back-and-forth transverse brushing action along the tangential direction of the gingival sulcus curve of the gingival sulcus position.

Second brushing action: the brushing action is performed by the bristle module facing the tooth surface positional scribing.

The bristle module facing the tooth surface positional scribing comprises a tooth surface positional scribing of each tooth on the jaw side of the upper dentition, a tooth surface positional scribing of each tooth on the cheek side of the upper dentition, a distal molar distal and middle surface positional scribing of the upper dentition, a tooth surface of each tooth on the lingual side of the lower dentition, a tooth surface of each tooth on the cheek side of the lower dentition and a a distal molar distal and middle surface positional scribing of the lower dentition. A soft and elastic bristle is adopted for bristle module in the tooth surface positional scribing, the flocking density is increased, dental plaque attached on the tooth surface can be effectively cleaned, and enamel is not damaged. Under the driving of the actuation module, the bristle module corresponding to the tooth surface part is made to brush along the tooth surface part from the position close to the gingiva to the tooth tip direction.

Third brushing action: the brushing action is performed by the bristle module facing the diastema positional scribing.

The bristle module facing the diastema positional scribing corresponds to the diastema between each tooth of the user and comprises the diastema between each tooth on the jaw side of the upper dentition, the diastema between each tooth on the cheek side of the upper dentition, the diastema between each tooth on the lingual side of the lower dentition and the diastema between each tooth on the cheek side of the lower dentition.

The bristle materials of the scribing bristle module in the diastema positional scribing are mixed and matched, namely: a soft and elastic sharpening fine bristle is adopted at the end part close to the alveolar ridge gingival mastoid positional scribing, so that the discomfort caused by mechanical stimulation to the alveolar ridge gingival mastoid positional scribing is avoided as much as possible, and the gingiva is protected. A spiral bristle with good elasticity and high toughness is adopted below the end part, so that the spiral bristle can easily extend into a tooth gap to remove stains such as plaque or food residues. Also, the bristle length of the diastema may be longer than that of other bristle modules depending on the size of the diastema. Under the driving of the actuation module, the bristle module corresponding to the diastema position is brushed along the crevice of the diastema position from the end part of the diastema close to the alveolar ridge gingival mastoid to the direction of the empty apical part.

Fourth brushing action: the brushing action is performed by the bristle module facing the occlusal surface positional scribing.

The bristle module facing the occlusal surface positional scribing, corresponding to the occlusal surface of each rear tooth of the user, comprises an occlusal surface positional scribing of the upper left 4 - upper left 8 of the upper dentition, an occlusal surface positional scribing of the upper right 4 - upper right 8 of the upper dentition, an occlusal surface positional scribing of the upper left 4 - upper left 8 of the lower dentition and an occlusal surface positional scribing of the upper right 4 - upper right 8 of the lower dentition. The bristle module facing the occlusal surface positional scribing adopts spiral bristles which are good in elasticity and strong in toughness. Under the driving of the actuation module, the bristle module corresponding to eachocclusal surface position is made to brush back and forth along the horizontal direction of the meshing surface part.

The correlation between dentition positional scribings and mouthpiece groove operation scribings is not necessarily one-to-one correspondence. Such as: a groove occlusal surface operation scribing corresponds to each occlusal surface positional scribing of the dentition. Each gingival sulcus positional scribing of the dentition can be provided with a plurality of cooresponding groove gingival sulcus operation scribings so as to realize tangential brushing operation. Because the gingival sulcus is a curve, two or more (such as three) gingival sulcus operation scribings are required to correspond to each gingival sulcus positional scribing on the groove so as to realize the fitting of a plurality of straight line segments to the gingival sulcus curve. Therefore, the requirements of the gingival sulcus tangential brushing operation by the Bass method are met. One or more corresponding operation scribings may be provided for scribings other than the gingival sulcus positional scribings.

Alternatively, at least two (for example, three) bristle modules are correspondingly arranged on each gingival sulcus operation scribing, the brushing direction of each bristle module is different, and therefore the arched curve of the gingival sulcus position can be better fitted.

A more preferred embodiment is provided that: aiming at the gingival sulcus position, each bristle module is set to be controlled to brush by two actuation modules, the two actuation modules alternately control the bristle modules to brush alternately along the tangential direction of the gingival sulcus curve and the normal direction of the gingival sulcus curve. Aiming at other positions of the dentition, each bristle module is set to be controlled to brush by one actuation module.

Step S5-A-3: establishing an excitation signal system of the three-dimensional mouthpiece model according to an actuation module system of the three-dimensional mouthpiece model.

In step S5-A-3, after an actuation module system of the three-dimensional mouthpiece model is established, an excitation signal source for controlling the actuation module to generate mechanical motion is arranged inside the mouthpiece model, and excitation signal data of the excitation signal source is established according to actuation requirement data of each actuation module, wherein the excitation signal source and the excitation signal data attached thereto form the excitation signal system of the mouthpiece model.

One or more excitation signal sources can be arranged, and when the excitation signal sources are arranged into a plurality, the power amplitudes of the excitation signal sources can be different. For example, the excitation signal sources with the same power amplitude are arranged for cleaning all gingival sulcus positions, the excitation signal sources with the same power amplitude are arranged for cleaning all tooth surface parts, and the like. For the positions with basically the same brushing force requirement, the excitation signal sources with the same power amplitude are arranged.

In order to realize the asymmetric cleaning operation requirements of the back and forth brushing force of the tooth surface positional scribing and the diastema positional scribing, the waveform design is preferably carried out on the alternating excitation signal, so that the rising edge of the signal is steeper, and the falling edge is milder.

Taking the tooth surface brushing as an example, a brushing cycle is set to brush from the part close to the gingival sulcus to the apical part, which is defined as the process of going forward, and then to brush back from the apical part to the gingival sulcus, which is defined as the process of going back. Due to the fact that different excitation signals are applied to the excitation sources, the speed of going forward is high, and the speed of going back is low. As a result, the bristles do not quickly stick to the gingival sulcus, which results in poor user experience
In order to realize the difference between the rising d and the falling edge of the signal in one period, the direct-current voltage signal with the corresponding voltage value can be generated by controlling the duty ratio of the PWM signal at the excitation signal source. For an actuation module corresponding to a bristle unit on the diastema operation scribing and the tooth surface operation scribing, the alternating excitation signal is asymmetric within one period. For example, a rising edge reaches a peak voltage from zero within 150ms, and the peak voltage is kept for 50ms; the falling edge takes 750 ms to gradually drop from the peak voltage to zero and remains at zero voltage for 50 ms. For the actuation module corresponding to the bristle module on the gingival sulcus operation scribing, the alternating excitation signal is symmetrical in one period. The rising edge reaches the peak voltage from zero in 350 ms, and the peak voltage is kept for 50ms, the falling edge gradually drops from peak voltage to zero for 350 ms and is held at zero voltage for 50 ms.

The dentition positional scribing system, the groove operation scribing system, the bristle module system, the actuation module system and the excitation signal system have sequentially corresponding mapping relationships, and the mapping relationships enable a latter action requirement to be determined on the basis of a former action requirement.

In order to fully meet the requirements of the Bass method, different types of materials can be adopted by each bristle module according to each position of the user dentition corresponding to the bristle module, and the bristle modules have different action directions. A dentifrice shell portion has hundreds (e.g., 200-800) of bristle modules on the inner wall of the phatnoma. On each bristle module, several bristles (e.g., 10-100) are implanted.

The steps S5-A-1 to S5-A-3 complete the establishment of a digitalized three-dimensional mouthpiece model, wherein the establishment not only comprises a static spatial layout, but also comprises a spatial layout of a groove position, a bristle module, an actuation module and an excitation source, and it further comprises a dynamic cleaning data layout, namely the action requirements of the bristle module, the actuation module and the excitation source signals respectively.

Finally, according to the established digital three-dimensional mouthpiece model, a finished mouthpiece for wearing by the user is manufactured.

### Embodiment 2

In this embodiment, the tooth cleaning is performed in cleaning operation mode II. The cleaning operation mode II comprises that: a conduit is embedded in the mouthpiece, one end of the conduit is connected with an excitation source, a bulge is arranged on the wall surface of the conduit, the bulge is a hollow tube communicated with a conduit channel, and the excitation source generates alternating excitation signals to control the bulge to perform reciprocating straightening and bending actions so as to clean relevant parts of teeth.

The occlusion positioning area and the occlusion detection area are calibrated in advance, the dentition position system and the groove operation scribing system are established, and the cleaning operation system of the cleaning system needs to be established according to the groove operation scribing system.

In the embodiment, the process for establishing the cleaning operation system specifically comprises:
Step S5-B-1: establishing a bulge module system of the three-dimensional mouthpiece model according to the groove operation scribing system of the three-dimensional mouthpiece model.
Step S5-B-1 specifically comprises: after a groove operation scribing system of the three-dimensional mouthpiece model is established, arranging the bulge on each groove operation scribing, and establishing bulge spatial layout data and brushing requirement data of each bulge module according to spatial layout data and operation requirement data of each groove operation scribing and spatial layout data of each corresponding dentition positional scribing, and all the bulge modules and data attached thereto form the bulge module system of the mouthpiece model.

The brushing requirement data for the bump module in this embodiment is exemplified as follows:
The brushing requirement data of each bulge in the bulge module of the gingival sulcus operation scribing is preset as follows: the static inclination angle between the bulge axis and the inner tooth surface of the gingival sulcus is 45 degrees; the bulge motion mode is a reciprocating symmetrical periodic motion, and the bulge motion period is 800ms; the bulge action direction is the tangential direction of the gingival sulcus arch arc curve, and the included angle is less than 15 degrees; the amplitude of the bulge motion is 2 mm run in the gingival sulcus.

The brushing requirement data of each bulge in the bulge module of the diastema operation scribing are preset as follows: the static inclination angle between the bulge axis and the diastema is 90 degrees; the bulge motion mode is a reciprocating asymmetric periodic motion, and the bulge motion period is 1000 ms; the bulge action direction is as follows: brushing from the crest end of the alveolar ridge of the diastema to the direction of the cusp, and returning to the direction of the crest end of the alveolar ridge from the direction of the cusp; the action time from the apical brush of the alveolar ridge to the apical direction is 200 ms, and the action time from the apical direction back to the apical direction of the alveolar ridge is 800 ms; the amplitude of the bump motion is a 60-degree swing angle.

Step S5-B-2: establishing a conduit system of the three-dimensional mouthpiece model according to the bulge module system of the three-dimensional mouthpiece model.

In step S5-B-2, after a bulge module system of the three-dimensional mouthpiece model is established, a conduit for conveying an excitation signal to each bulge on each bulge module is arranged in a mouthpiece of the mouthpiece model and comprises a main conduit and each branch conduit, conduit spatial layout data of each conduit is established according to spatial layout data and brushing requirement data of each bulge module, and all of the conduits and data attached thereto form the conduit system of the mouthpiece model.

The dentition positional scribing system, the groove operation scribing system, the bulge module system and the conduit system have sequentially corresponding mapping relationships.

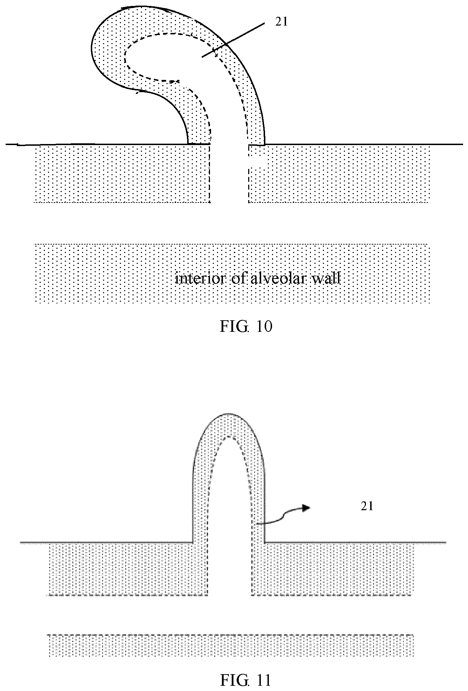

Referring to FIGs. 10 and 11, the working principle of cleaning using the bulges of the present embodiment is as follows:
Under the normal state (*i.e.* when the pump module does not perform pumping action), the bulge is shaped by preset stress according to the requirements of the bulge module system due to the material of the bulge, and the bulge is curved in the preset direction as shown in FIG. 10; the pump module can be connected with one or more air inlets or a water storage tank, gas or liquid is continuously conveyed to the main conduit under the pumping action of the pump module in the handle, the main conduit is communicated with each branch conduit, and then power is transmitted to each branch conduit. A semi-hollow tube 21 communicated with the branch conduits is arranged on the wall of each branch conduit, and power is transmitted to the tube 21. Since the inner air pressure of the tube 21 is greater than the outer atmospheric pressure, the tube 21 has a bulging straight shape as shown in FIG. 11, and the tube 21 bulges upward when the pressure in the main and branch conduits increases, and contracts when the pressure decreases. When the pump module reciprocates to perform a pumping action, the semi-hollow tube 21 reciprocates to perform a straightening and bending action, so that a friction action with a predetermined direction and amplitude is generated between the semi-hollow tube 21 and the tooth surface, and teeth are cleaned.

### Embodiment 3

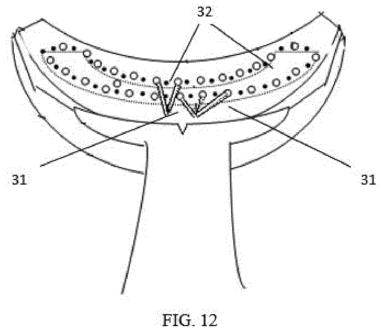

Referring now to FIG. 12, in one embodiment, tooth cleaning is accomplished using cleaning operation mode III. The cleaning operation mode III is as follows: a plurality of conduits 31 are embedded in the mouthpiece, one end of each conduit 31 is connected with an excitation source, the other end of each conduit 31 is provided with a small hole and corresponds to a relevant position of a tooth, and the excitation source sprays liquid, gas or vapor-liquid mixed mist to the relevant position of the tooth through the conduits and the small holes so as to clean the relevant position of the tooth.

Wherein, the occlusion positioning area and the occlusion detection area are calibrated in advance, the dentition position system and the groove operation scribing system are established, and the cleaning operation system of the cleaning system needs to be established according to the groove operation scribing system.

In the embodiment, the process for establishing the cleaning operation system specifically comprises:
Step S5-C-1: establishing a small hole opening system of the three-dimensional mouthpiece model according to the groove operation scribing system of the three-dimensional mouthpiece model.
Step S5-C-1 specifically comprises: after the groove operation scribing system of the three-dimensional mouthpiece model is established, arranging small hole openings on each groove operation scribing, and establishing spatial layout data of each small hole opening according to groove spatial layout data and operation requirement data of each groove operation scribing and dentition spatial layout data of each corresponding dentition positional scribing, wherein all the small hole openings and data attached thereto form the small hole opening system of the mouthpiece model, such as the aperture of the small hole.
Step S5-C-2: establishing a conduit system of the three-dimensional mouthpiece model according to the small hole opening system of the three-dimensional mouthpiece model, such as the number of conduits, the inner diameter of the conduits and the like.

In step S5-C-2, the method further comprises, after the small hole opening system of the three-dimensional mouthpiece model is established, a conduit for conveying excitation signals to each small hole opening is arranged in the phatnoma of the mouthpiece model and comprising a main conduit and each branch conduit, the spatial layout data of each conduit according to the spatial layout data of each small hole opening is established, and all the conduits and data attached thereto form the conduit system of the mouthpiece model, the excitation source spraying liquid, gas or gas-liquid mixed mist to a relevant position of the tooth through the conduit and the small hole opening so as to clean the relevant position of the tooth. The small hole formed in the conduit should correspond to the opening small hole formed in the groove operation scribing.

The dentition positional scribing system, the groove operation scribing system, the small hole opening system and the conduit system have sequentially corresponding mapping relationships.

The working principle of cleaning by adopting the conduit and the small holes in the embodiment is as follows:
A plurality of small hole openings 32 are formed in relevant parts of the groove of the mouthpiece, the small hole openings 32 preferably correspond to interdental seams of teeth of a user, one ends of the plurality of small holes 32 are connected with conduits 31 embedded in the mouthpiece, one ends of the conduits 31 are also provided with small holes and correspond to the positions of the small hole openings 32 in the groove, so that the conduits 31 are communicated with the small hole openings 32. The other ends of the plurality of conduits 31 extend into the handle and are connected with the pump module through a main conduit, and liquid, gas or mist is sprayed out of the small hole openings 32 under the pumping action of the pump module to clean the crevices.

Finally, according to different cleaning operation modes, a digitalized three-dimensional mouthpiece model is established, and a finished mouthpiece for wearing by the user is manufactured.

Specifically, the user's dentifrice shell portion is made according to the user's aforementioned dentifrice shell portion design. Taking the selected cleaning operation mode I as an example, the manufacturing content comprises an inner framework, an outer coating layer, an actuation module, a bristle module and the like of a mouthpiece portion, and a 3D printing process, a flocking process and the like are specifically used in the manufacturing method. Taking the selected cleaning operation mode II as an example, the manufacturing content comprises an inner framework, an outer coating, a conduit, a bulge and the like of the mouthpiece portion. Moreover, a pressure sensor and a signal transmission wire thereof are embedded in the groove of the mouthpiece part. The embedded part of the pressure sensor is the inside of the groove corresponding to the "occlusion detection area" of the surface of the groove. To facilitate handling by the user, a handle portion is also formed to mate with the shell, the handle having a space therein for receiving an excitation source and other auxiliary components, thereby forming the shell dentifrice of the user.

After the finished mouthpiece is manufactured, the user wears the finished mouthpiece to carry out correlation detection in occlusion process, wherein the detection process specifically comprises:
detecting whether a pressure value generated by abutting the occlusion detection area of a mouthpiece groove and the occlusion positioning area of a user dentition reaches a set threshold value and whether a duration time reaches a set time interval; and
after detecting that the pressure value reaches the threshold value and the duration time reaches the set time interval, triggering the excitation module to generate the alternating excitation signal, and then controlling a generation of a cleaning operation.

In particular, the user places the dentifrice shell portion into the oral cavity such that the upper and lower dentitions are aligned with the upper and lower grooves of the shell portion, initiating an occlusal action, with the "occlusal locating areas" of the upper and lower dentitions of the user abutting the "occlusal detection areas" of the upper and lower phatnomas of the shell, respectively, at which point the "user upper dentition - mouthpiece upper groove spatial relation layout" is consistent with the "the upper dentition positional scribing array - upper groove operation scribing array marking spatial relation layout", and the "user lower dentition - mouthpiece lower groove spatial relation layout" is consistent with the "the lower dentition positional scribing array - the lower groove operation scribing array marking spatial relation layout".

Then whether the pressure value generated by occlusion reaches a set threshold value and lasts for a set time interval is detected. Due to the fact that the pressure sensor is installed in the phatnoma, after the pressure sensor detects the pressure, the signal is sent to the processor to judge whether the pressure value meets the standard or not.

When the pressure value reaches a threshold value and the duration exceeds a set time interval (for example, exceeds 500 milliseconds), an excitation source is triggered to generate an alternating excitation signal, and then the cleaning operation and the cleaning action generation are controlled. When it is detected that the pressure value has not reached a threshold value or has not reached a set time interval (for example, not exceeding 500 milliseconds), the cleaning operation and the cleaning action are controlled to stop.

In operation mode I, after an excitation signal is generated by an excitation source, the actuation module starts to do reciprocating motion so as to drive the bristle module to perform reciprocating brushing on various parts of teeth; in operation mode II, when the excitation source intermittently generates excitation signals, the pressure in the main conduit and each branch conduit is triggered to increase and decrease, so that the pressure in the bulge increases and decreases, and the bulge carries out reciprocating telescopic motion to brush each position of the tooth; in operation mode III, when an excitation signal is generated intermittently by the excitation source, the conduit is triggered to inject gas, liquid or mist outwards through the opening small hole so as to clean various parts of teeth. Due to the fact that the user teeth are subjected to mold taking and positional scribing in advance, individual differences exist in the amplitude, the direction, the frequency and even the selection of bristle materials according to different operation areas.

In order to more conveniently illustrate the correlation establishing process of the present invention, an embodiment of cleaning of gingival sulcus position and diastema position will now be described by way of example from the entire dynamic change of "cleaning demand data - operation demand data - brushing demand data - actuation demand data - excitation signal data".

Initial cleaning requirement parameters for gingival sulcus positional scribing: the corresponding bristle type is a sharpened fine bristle, the diameter parameter of the bristle material is 0.01-0.02 mm, and the static inclination angle between the bristle and the inner tooth surface of the gingival sulcus is 45 degrees. The action mode of the bristle is a reciprocating symmetrical periodic motion, and the action period of the bristle is 800 ms. The included angle between the action direction of the bristle and the tangential direction of the arch curve of the gingival sulcus is less than 15 degrees, and the action amplitude of the bristle is 1mm run in the gingival sulcus. Initial cleaning requirement parameters for diastema positional scribing: the corresponding bristle type is a flexible spiral bristle, the diameter parameter of the bristle material is 0.02-0.03 mm, the static inclination angle between the bristle and the diastema is 90 degrees, the action mode of the bristle is a reciprocating asymmetric periodic motion, and the action period of the bristle is 1000 ms. The action directions of the bristles are as follows: brushing from the apical end of the alveolar ridge of the diastema to the apical direction, and returning from the apical direction to the apical direction of the alveolar ridge. The action time from the apical brush of the alveolar ridge to the apical direction is 200 ms, and the action time from the apical direction back to the apical direction of the alveolar ridge is 800 ms. The brush action amplitude is a 60-degree swing angle.

The initial cleaning requirement parameter gives the initial value range of the default state of scribing cleaning requirement data of various parts. Most of the parameters in the cleaning demand data may be consistent with the values in the initial cleaning demand parameters. The operation requirement data of the groove operation scribing receives the cleaning requirement data of the corresponding dentition positional scribings, which is not a value range, but a specific numerical value of the operation requirement data of each groove operation scribing established according to the spatial layout data of each groove operation scribing and the cleaning requirement data of each corresponding dentition positional scribings. The brushing requirement data of the bristle modules in the mouthpiece groove operation scribing receive the operation requirement data of the corresponding groove operation scribing, and the spatial layout data and the brushing requirement data of each bristle module are established according to the spatial layout data and the operation requirement data of each groove operation scribing. Actuation requirement data of the actuation modules in the mouthpiece groove operation scribing receive brushing requirement data of the bristle modules in the corresponding mouthpiece groove operation scribing, and corresponding actuation requirement data are established according to the spatial layout data and the brushing requirement data of the bristle modules and the spatial layout data of the corresponding actuation modules. The excitation signal data in the mouthpiece receives the actuation requirement data of the actuation modules in the mouthpiece groove operation scribing, and the excitation signal data of the excitation signal source is established according to the actuation requirement data of each actuation module.

An example of operation requirement data in a mouthpiece groove operational scribing is as follows. Operation requirement data of gingival sulcus operation scribing: the type of bristle is sharpened fine bristle, the diameter parameter of bristle is 0.01 mm, and the static inclination angle between bristle and inner tooth surface of gingival sulcus is 45 degrees; the bristle motion mode is a back-and-forth symmetrical periodic motion, and the bristle motion period is 800 ms; the included angle between the action direction of the bristle and the tangential direction of the arch curve of the gingival sulcus is less than 15 degrees, and the action amplitude of the bristle is 1 mm run in the gingival sulcus. The operation requirement data of the diastema operation scribing are as follows: the bristle type is flexible spiral bristle, the diameter parameter of bristle material is 0.02 mm, and the static inclination angle between bristle and diastema is 90 degrees; the bristle motion mode is a back-and-forth asymmetric periodic motion, and the bristle motion period is 1000 ms; the action directions of the bristles are as follows: brushing from the crest end of the alveolar ridge of the diastema to the direction of the cusp, and returning to the direction of the crest end of the alveolar ridge from the direction of the cusp; the action time from the apical brush of the alveolar ridge to the apical direction is 200 ms, and the action time from the apical direction back to the apical direction of the alveolar ridge is 800 ms. The brush action amplitude is a 60-degree swing angle.

An example of brushing demand data for a bristle module in a mouthpiece groove operation scribing is as follows. Brushing requirement data of a bristle module corresponding to gingival sulcus operation scribing: the type of bristle is sharpened fine bristle, the diameter parameter of bristle is 0.01mm, and the static inclination angle between bristle and inner tooth surface of gingival sulcus is 45 degrees; the bristle motion mode is a back-and-forth symmetrical periodic motion, and the bristle motion period is 800 ms; the included angle between the action direction of the bristle and the tangential direction of the arch curve of the gingival sulcus is less than 15 degrees, and the action amplitude of the bristle is 1mm run in the gingival sulcus. Brushing requirement data of a bristle module for diastema operation scribing: the bristle type is flexible spiral bristle, the diameter parameter of bristle material is 0.02 mm, and the static inclination angle between bristle and diastema is 90 degrees; the bristle motion mode is a back-and-forth asymmetric periodic motion, and the bristle motion period is 1000 ms; the action directions of the bristles are as follows: brushing from the crest end of the alveolar ridge of the diastema to the direction of the cusp, and returning to the direction of the crest end of the alveolar ridge from the direction of the cusp; the action time from the apical brush of the alveolar ridge to the apical direction of the crown is 200ms, and the action time from the apical direction to the apical direction of the alveolar ridge is 800ms; the brush action amplitude is a 60-degree swing angle.

Examples of actuation requirement data for an actuation module in a mouthpiece groove operation scribing are as follows. Actuation requirement data of the actuation module corresponding to the gingival sulcus operation scribing are as follows: the mechanical motion mode is a back-and-forth symmetrical periodic motion, and the mechanical motion period is 800 ms; the mechanical motion amplitude is 1 mm run. Actuation requirement data of the actuation module corresponding to the diastema operation scribing are as follows: the mechanical motion mode is a back-and-forth asymmetric periodic motion, and the mechanical motion period is 1000 ms; the mechanical motion time is 200 ms for contraction time and 800 ms for relaxation time; the mechanical motion amplitude is 2 mm.

The excitation signal data in the mouthpiece is exemplified as follows. For an actuation module on a gingival sulcus operation scribing, the alternating excitation signal of the actuation module is symmetrical within one period, the rising edge reaches a peak voltage of 16V from zero within 350 ms, and the peak voltage is kept for 50 ms; the falling edge is ramped from peak voltage 16 V to zero for 350 ms and is held at zero voltage for 50 ms. For the actuation modules on the diastema operation scribing and tooth surface operation scribing, the alternating excitation signal is asymmetric for one cycle, the rising edge reaches a peak voltage of 16 V from zero in 150 ms, and remains at the peak voltage for 50 ms, while the falling edge takes 750 ms to gradually drop from the peak voltage of 16 V to zero and remains at zero voltage for 50 ms.

According to the mouthpiece-type teeth-cleaner of the invention disclosed by the embodiment of the invention, the correlation between the tooth model and the mouthpiece model and the correlation between the tooth model and the cleaning system on the mouthpiece model are established, so that a user can carry out omnibearing automatic and personalized cleaning when wearing the mouthpiece.

Compared with the prior art, the mouthpiece-type teeth-cleaner disclosed by the embodiment of the invention has the advantages that a user does not need to move the cleaning device everywhere in the oral cavity, can effectively clean different parts of teeth at the same time only by performing occlusion and releasing actions. The cleaner is very convenient to use, can well clean the tooth parts on the lingual side, and can perform personalized cleaning on the different parts of the teeth. Better implementation of Bass method is achieved.

Finally, it should be noted that: the embodiments are only intended to illustrate the technical solution of the present invention, but not to limit it.

## Claims

1. A mouthpiece-type teeth cleaner, comprising:
conduits (31) embedded in the mouthpiece, wherein one end of the conduits (31) is provided with small hole openings (32);
the teeth cleaner further comprises
an excitation source, configure to generate one or more groups of alternating excitation signals,
wherein one or more cleaning operations, with different cleaning actions applied to various parts of the teeth are generated according to the excitation signals;
a bristle module (61, 62) arranged on the mouthpiece;
a hollow bulge (21) communicated with a channel of the conduits (31) and arranged on a wall surface of the conduits (31); and
a plurality of actuation modules connected with the excitation source and arranged in the mouthpiece;
wherein,
said one end of the conduits (31) is corresponding to a relevant position of the tooth;
another end of the conduits (31) is connected with the excitation source; and
in the mouthpiece, a correlation between the cleaning operation and the cleaning action and each part of applied tooth is established in advance, and the correlation enabling each position of the tooth of the user to be individually cleaned.

2. The teeth cleaner according to claim 1, wherein:
the correlation is established by the mouthpiece being worn to perform correlation detection in occlusion process and trigger generating of an excitation signal;
the mouthpiece is manufactured according to a digital three-dimensional mouthpiece model; and
the digital three-dimensional mouthpiece model is established according to a three-dimensional tooth model, and is matched with the three-dimensional tooth model, wherein a cleaning system of the three-dimensional mouthpiece model and each part of teeth of the three-dimensional tooth model having corresponding spatial relation layout; and
the three-dimensional tooth model is established according to a spatial layout image of an oral cavity of a user.

3. The teeth cleaner according to claim 2, wherein:
a cleaning operation system of the cleaning system is established according to a groove operation scribing system of the three-dimensional mouthpiece model;
the groove operation scribing system is established according to a dentition positional scribing system of the three-dimensional tooth model; and
the dentition positional scribing system is established by scribing an outer surface of dentition of the three-dimensional tooth model according to the three-dimensional tooth model of a selected occlusion positioning area,
wherein occlusion detection areas on corresponding parts of upper groove and lower groove of the three-dimensional mouthpiece model are marked according to the occlusion positioning area, and
wherein the occlusion positioning area is one or more selected positions on upper dentition and lower dentition of the three-dimensional tooth model.

4. The teeth cleaner according to claim 3, wherein said the dentition positional scribing system established by scribing the outer surface of dentition of the three-dimensional tooth model according to the three-dimensional tooth model of the selected occlusion positioning area comprises:
the dentition positional scribing system of the tooth model is consisting of a plurality of mutually connected dentition positional scribings, and dentition spatial layout data and cleaning requirement data of each of the dentition positional scribings;
the plurality of the dentition positional scribings are established by scribing surface area, except the occlusion positioning area, of the outer surface of the dentition of the model according to the three-dimensional tooth model in selected occlusion positioning area, wherein different initial cleaning requirement parameters are preset for positional scribings of different classes in advance, and the initial cleaning requirement parameters comprise selection of cleaning operation modes;
the dentition spatial layout data and cleaning requirement data of the dentition positional scribings are established according to image information of the dentition positional scribings and the initial cleaning requirement parameters of the dentition positional scribings.

5. The teeth cleaner according to claim 4, wherein said the groove operation scribing system is established according to a dentition positional scribing system of the three-dimensional tooth model comprises:
the groove operation scribing system is consisting of a plurality of groove operation scribings, and groove spatial layout data and operation requirement data of each of the plurality of groove operation scribings;
each of the operation requirement data is established according to the spatial layout data of each of the groove operation scribings, the spatial layout data of each of the dentition positional scribings and the cleaning requirement data corresponding to the selected cleaning operation mode; and
each of the plurality of groove operation scribings are established by marking the groove operation scribings at corresponding parts of the surface area, except the occlusion detection area, of the surface of on the upper groove and the lower groove of the three-dimensional mouthpiece model, according to the corresponding relation with the upper dentition and the lower dentition of the tooth model, and according to the dentition spatial layout data of each dentition positional scribing and the cleaning requirement data corresponding to selected cleaning operation mode;
wherein a gap existing between each groove operation scribing and each corresponding dentition positional scribing when the occlusion detection area abuts against the occlusion positioning area by means of the groove spatial layout data.

6. The teeth cleaner according to claim 5, wherein,
according to different cleaning requirements of each position of the tooth, the dentition positional scribings are classified into categories comprising: gingival sulcus positional scribing, tooth surface positional scribing, diastema positional scribing, tooth occlusal surface positional scribing, tooth section positional scribing and distal molar distal and middle surface positional scribing; and
according to the scribing type of the dentition positional scribing, the groove operation scribing is divided into corresponding categories comprising: gingival sulcus operation scribing, tooth surface operation scribing, diastema operation scribing, tooth occlusal surface operation scribing, tooth section operation scribing and distal molar distal and middle surface operation scribing.

7. The teeth cleaner according to any one of claims 2 to 6, wherein the cleaning operation is generated by the cleaning system, and the cleaning operation mode is based on one or more combinations of the following three modes:
operation mode I:
the actuation modules receiving the excitation signal and generating one or more groups of mechanical motions; and
the mechanical motion driving the bristle module (61, 62) arranged on the mouthpiece to perform reciprocating brushing so as to clean relevant parts of teeth;
operation mode II:
the excitation source generating alternating excitation signals to control the bulge to perform reciprocating straightening and bending actions so as to clean relevant parts of teeth;
operation mode III:
the excitation source spraying liquid, gas or vapor-liquid mixed mist to relevant position of the tooth through the conduits (31) and the small hole openings (32) so as to clean the relevant position of the tooth.

8. The teeth cleaner according to claim 7, wherein when the cleaning operation is carried out in operation mode I,
an excitation signal system of the three-dimensional mouthpiece model is established according to an actuation module system of the three-dimensional mouthpiece model;
the actuation module system of the three-dimensional mouthpiece model is established according to a bristle module system of the three-dimensional mouthpiece model;
the bristle module system of the three-dimensional mouthpiece model is established according to the groove operation scribing system of the three-dimensional mouthpiece model;
wherein, the dentition positional scribing system, the groove operation scribing system, the bristle module system, the actuation module system and the excitation signal system having sequentially corresponding mapping relationships,
preferably,
the excitation signal system consists of the excitation signal source and excitation signal data of the excitation signal source, wherein the excitation signal source is arranged inside the mouthpiece model for controlling the actuation module to generate mechanical motion, and the excitation signal data of the excitation signal source is established according to actuation requirement data of each actuation module,
the actuation module system consists of the actuation modules, and bristle spatial layout data and the brushing requirement data of each of the actuation modules, wherein each of the actuation modules is arranged in phatnoma of the mouthpiece model and drives each bristle module (61, 62) to act, and the actuation spatial layout data and the actuation requirement data of each actuation module is established according to the bristle spatial layout data and the brushing requirement data of each bristle module (61, 62),
the bristle module system consists of the bristle modules (61, 62), and groove spatial layout data and operation requirement data of each of the bristle modules (61, 62), wherein each of the bristle modules (61, 62) is arranged on each groove operation scribing, and the bristle spatial layout data and the brushing requirement data of each bristle module (61, 62) are established according to groove spatial layout data and operation requirement data of each groove operation scribing and dentition spatial layout data of each corresponding dentition positional scribing.

9. The teeth cleaner according to claim 7, wherein when the cleaning operation is carried out in operation mode II,
a conduit system of the three-dimensional mouthpiece model is established according to a bulge module system of the three-dimensional mouthpiece model; and
the bulge module system of the three-dimensional mouthpiece model is established according to the groove operation scribing system of the three-dimensional mouthpiece model;
wherein, the dentition positional scribing system, the groove operation scribing system, the bulge module system and the conduit system having sequentially corresponding mapping relationships;
preferably,
the conduit system consists of the conduits (31) and the conduit spatial layout data of each of the conduits (31), wherein the conduits (31) are arranged in the phatnoma of the mouthpiece model, for conveying an excitation signal to each bulge on each bulge module, and comprising a main conduit and each branch conduit, and the conduit spatial layout data of each conduit (31) is established according to spatial layout data and brushing requirement data of each bulge module;
the bulge module system consists of the bulge modules, and bulge spatial layout data and brushing requirement data of each of the bulge modules, wherein the bulge spatial layout data and the brushing requirement data of each bulge module is established according to spatial layout data and operation requirement data of each groove operation scribing and spatial layout data of each corresponding dentition positional scribing; and
wherein, the excitation signal is an alternating air pressure signal, a hydraulic pressure signal or a voltage signal.

10. The teeth cleaner according to claim 7, wherein when the cleaning operation is carried out in operation mode III,
a conduit system of the three-dimensional mouthpiece model is established according to a small hole opening system of the three-dimensional mouthpiece model; and
the small hole opening system of the three-dimensional mouthpiece model is established according to the groove operation scribing system of the three-dimensional mouthpiece model;
wherein, the dentition positional scribing system, the groove operation scribing system, the small hole opening system and the conduit system having sequentially corresponding mapping relationships;
preferably,
the conduit system consists of conduits (31) and the spatial layout data of each conduit (31), wherein the conduits (31) is arranged in the phatnoma of the mouthpiece model, for conveying excitation signals to each small hole opening (32), and comprising a main conduit and each branch conduit, the spatial layout data of each conduit (31) is established according to the spatial layout data of each small hole opening;
the small hole opening system consists of the small hole openings (32) and spatial layout data of each small hole opening (32), wherein the small hole openings (32) are arranged on each groove operation scribing, and spatial layout data of each small hole opening (32) is established according to groove spatial layout data and operation requirement data of each groove operation scribing and dentition spatial layout data of each corresponding dentition positional scribing;
wherein the excitation source spraying liquid, gas or gas-liquid mixed mist to a relevant position of the tooth through the conduits (31) and the small hole openings (32) so as to clean the relevant position of the tooth; and
wherein, the excitation signal is an alternating air pressure signal or a hydraulic pressure signal.

11. The teeth cleaner according to any one of claims 7 to 10, wherein, each gingival sulcus positional scribing is set to correspond to at least two of the gingival sulcus operation scribings, or each gingival sulcus operation scribing is set to correspond to at least two of the bristle modules (61, 62) to be arranged on;
and/or
for a gingival sulcus position, each bristle module (61, 62) is set to be controlled to brush by two of the actuation modules which alternately control the bristle module (61, 62) to brush in a tangential direction of a gingival sulcus curve and a normal direction of the gingival sulcus curve alternately.

12. The teeth cleaner according to any one of claims 7 to 11, wherein the excitation signal is an alternating air pressure signal, a hydraulic pressure signal or a voltage signal.

13. The teeth cleaner according to any one of claims 7 to 12, wherein the actuation module is composed of a driving unit and a transmission unit, wherein the driving unit is connected with the excitation source and receives an excitation signal generated by the excitation source and drives the transmission unit to act, and the transmission unit is connected with the bristle module (61, 62) so as to drive the bristle module (61, 62) to act in a preset manner,
preferably, the driving unit is a pneumatic driving unit, a hydraulic driving unit or a voltage driving unit; and the transmission unit is a 7-shaped connecting rod or a T-shaped supporting rod, wherein the 7-shaped connecting rod comprising a first cross rod and a first vertical handle which are integrally formed, the T-shaped supporting rod comprising a second cross rod and a second vertical handle which are integrally formed.

14. The teeth cleaner according to any one of claims 7 to 12, wherein the actuation module is at least one artificial muscle (41, 71-72, 91-94), the bristle module (61, 62) is composed of a flocking tray (61) and one bristle (62) or a cluster of bristles (62) fixed on the flocking tray (61), one end of the artificial muscle (41, 71-72, 91-94) is connected with the flocking tray (61), the excitation source is a voltage signal, and the excitation source controls the artificial muscle (41, 71-72, 91-94) to contract and move by applying a voltage signal to the artificial muscle (41, 71-72, 91-94) so as to drive the tray to move,
preferably, the at least one artificial muscle (41, 71-72, 91-94) is provided in at least two (71-72, 91-94), and for the gingival sulcus position, one bristle module (61, 62) is set to be controlled to brush by the at least two artificial muscles (71-72, 91-94), the artificial muscles (71-72, 91-94) are arranged around the flocking tray (61) of the bristle module (61, 62), the artificial muscles (71-72, 91-94) are set to be driven by a plurality of sets of excitation signals with phase differences, the plurality of sets of excitation signals enable part of the artificial muscles (71-72, 91-94) to contract by cooperation of different voltage phase differences, and the bristle module (61, 62) is driven to move towards a contraction direction of the artificial muscle (71-72, 91-94).

15. The teeth cleaner according to any one of claims 2 to 14, wherein in the correlation detection,
the alternating excitation signals is triggered by an excitation module detecting that a pressure value generated by abutting the occlusion detection area of a mouthpiece groove and the occlusion positioning area of a user dentition reaches the threshold value and the duration time reaches the set time interval.

## Patentansprüche

1. Zahnreiniger vom Mundstücktyp, umfassend:
Rohrleitungen (31), die in das Mundstück eingebettet sind, wobei ein Ende der Rohrleitungen (31) mit kleinen Lochöffnungen (32) ausgestattet ist;
wobei der Zahnreiniger des Weiteren umfasst:
eine Anregungsquelle, die ausgestaltet ist, um eine oder mehrere Gruppen von alternierenden Anregungssignalen zu generieren,
wobei eine oder mehrere Reinigungsoperationen mit unterschiedlichen Reinigungsaktionen, die auf verschiedene Teile der Zähne angewendet werden, entsprechend den Anregungssignalen generiert werden;
ein Borstenmodul (61, 62), das auf dem Mundstück angeordnet ist;
eine hohle Ausbuchtung (21), die mit einem Kanal der Rohrleitungen (31) kommuniziert und auf einer Wandoberfläche der Rohrleitungen (31) angeordnet ist; und
eine Vielzahl von Betätigungsmodulen, die mit der Anregungsquelle verbunden und in dem Mundstück angeordnet sind; wobei
das eine Ende der Rohrleitungen (31) einer relevanten Position des Zahns entspricht;
ein anderes Ende der Rohrleitungen (31) mit der Anregungsquelle verbunden ist; und
in dem Mundstück eine Korrelation zwischen der Reinigungsoperation und der Reinigungsaktion und jedem Teil des angewendeten Zahns vorab erstellt wird, und die Korrelation ermöglicht, dass jede Position des Zahns des Benutzers individuell gereinigt wird.

2. Zahnreiniger nach Anspruch 1, wobei:
die Korrelation dadurch erstellt wird, dass das Mundstück getragen wird, um Korrelationsdetektierung in dem Gebissschlussprozess durchzuführen und das Generieren eines Anregungssignals auszulösen;
das Mundstück gemäß einem digitalen dreidimensionalen Mundstückmodell gefertigt wird; und
das digitale dreidimensionale Mundstückmodell gemäß einem dreidimensionalen Zahnmodell erstellt wird und mit dem dreidimensionalen Zahnmodell abgeglichen wird, wobei ein Reinigungssystem des dreidimensionalen Mundstückmodells und jedes Teils der Zähne des dreidimensionalen Zahnmodells ein entsprechendes räumliches Beziehungslayout haben; und
das dreidimensionale Zahnmodell gemäß einem räumlichen Layoutbild einer Mundhöhle eines Benutzers erstellt wird.

3. Zahnreiniger nach Anspruch 2, wobei:
ein Reinigungsoperationssystem des Reinigungssystems gemäß einem Furchenoperationsbeschreibungssystem des dreidimensionalen Mundstückmodells erstellt wird;
das Furchenoperationsbeschreibungssystem gemäß einem Bezahnungspositionsbeschreibungssystem des dreidimensionalen Zahnmodells erstellt wird; und
das Bezahnungspositionsbeschreibungssystem durch Beschreiben einer Außenoberfläche der Bezahnung des dreidimensionalen Zahnmodells gemäß dem dreidimensionalen Zahnmodell eines ausgewählten Gebissschlusspositionierungsbereichs erstellt wird,
wobei Gebissschlussdetektierungsbereiche auf entsprechenden Teilen der oberen Furche und unteren Furche des dreidimensionalen Mundstückmodells gemäß dem Gebissschlusspositionierungsbereich markiert werden, und
wobei der Gebissschlusspositionierungsbereich eine oder mehrere ausgewählte Positionen auf der oberen Bezahnung und der unteren Bezahnung des dreidimensionalen Zahnmodells ist.

4. Zahnreiniger nach Anspruch 3, wobei das Bezahnungspositionsbeschreibungssystem, das durch Beschreiben der Außenoberfläche der Bezahnung des dreidimensionalen Zahnmodells gemäß dem dreidimensionalen Zahnmodell des ausgewählten Gebissschlusspositionierungsbereichs erstellt worden ist, umfasst:
das Bezahnungspositionsbeschreibungssystem des Zahnmodells besteht aus einer Vielzahl von miteinander verbundenen Bezahnungspositionsbeschreibungen und räumlichen Bezahnungslayoutdaten und Reinigungsanforderungsdaten von jeder der Bezahnungspositionsbeschreibungen;
die Vielzahl der Bezahnungspositionsbeschreibungen wird durch Beschreiben des Oberflächenbereichs mit Ausnahme des Gebissschlusspositionsbereichs der Außenoberfläche der Bezahnung des Modells gemäß dem dreidimensionalen Zahnmodell in ausgewähltem Gebissschlusspositionsbereich erstellt, wobei unterschiedliche Anfangsreinigungsanforderungsparameter für Positionsbeschreibungen unterschiedlicher Klassen vorab festgelegt werden und die Anfangsreinigungsanforderungsparameter Auswahl von Reinigungsoperationsmodi umfasst;
die räumlichen Bezahnungslayoutdaten und Reinigungsanforderungsdaten der Bezahnungspositionsbeschreibungen werden gemäß Bildinformationen der Bezahnungspositionsbeschreibungen und der Anfangsreinigungsanforderungsparameter der Bezahnungspositionsbeschreibungen erstellt.

5. Zahnreiniger nach Anspruch 4, wobei das Furchenoperationsbeschreibungssystem, das gemäß einem Bezahnungspositionsbeschreibungssystem des dreidimensionalen Zahnmodells erstellt wird, umfasst:
das Furchenoperationsbeschreibungssystem besteht aus einer Vielzahl von Furchenoperationsbeschreibungen und räumlichen Furchenlayoutdaten und Operationsanforderungsdaten von jeder der Vielzahl von Furchenoperationsbeschreibungen;
jede der Operationsanforderungsdaten wird gemäß den räumlichen Layoutdaten von jeder der Furchenoperationsbeschreibungen, den räumlichen Layoutdaten von jeder der Bezahnungspositionsbeschreibungen und den Reinigungsanforderungsdaten entsprechend dem ausgewählten Reinigungsoperationsmodus erstellt; und
jede der Vielzahl der Furchenoperationsbeschreibungen wird durch Markieren der Furchenoperationsbeschreibungen an entsprechenden Teilen des Oberflächenbereichs mit Ausnahme des Gebissschlussdetektierungsbereichs von der Oberfläche auf der oberen Furche und der unteren Furche des dreidimensionalen Mundstückmodells gemäß der entsprechenden Beziehung mit der oberen Bezahnung und der unteren Bezahnung des Zahnmodells und gemäß den räumlichen Bezahnungslayoutdaten von jeder Bezahnungspositionsbeschreibung und den Reinigungsanforderungsdaten entsprechend dem ausgewählten Reinigungsoperationsmodus erstellt;
wobei zwischen jeder Furchenoperationsbeschreibung und jeder entsprechenden Bezahnungspositionsbeschreibung eine Lücke vorhanden ist, wenn der Gebissschlussdetektierungsbereich mittels der räumlichen Furchenlayoutdaten an dem Gebissschlusspositionsbereich anliegt.

6. Zahnreiniger nach Anspruch 5, wobei:
gemäß unterschiedlichen Reinigungsanforderungen von jeder Position des Zahns die Bezahnungspositionsbeschreibungen in Kategorien klassifiziert werden, umfassend: Zahnfleischsulkuspositionsbeschreibung, Zahnoberflächenpositionsbeschreibung, Diastemapositionsbeschreibung, Zahnokklusaloberflächenpositionsbeschreibung, Zahnschnittpositionsbeschreibung und distal-molar-distale und Mitteloberflächenpositionsbeschreibung; und
gemäß dem Beschreibungstyp der Bezahnungspositionsbeschreibung die Furchenoperationsbeschreibung in entsprechende Kategorien unterteilt wird, umfassend: Zahnfleischsulkuspositionsbeschreibung, Zahnoberflächenoperationsbeschreibung, Diastemaoperationsbeschreibung, Zahnokklusaloberflächenoperationsbeschreibung, Zahnschnittoperationsbeschreibung und distal-molar-distale und Mitteloberflächenoperationsbeschreibung.

7. Zahnreiniger nach einem der Ansprüche 2 bis 6, wobei die Reinigungsoperation durch das Reinigungssystem generiert wird und der Reinigungsoperationsmodus auf einer oder mehreren Kombinationen der folgenden drei Modi basiert:
Operationsmodus I:
die Betätigungsmodule empfangen das Anregungssignal und generieren eine oder mehrere Gruppen von mechanischen Bewegungen; und
die mechanische Bewegung treibt das Borstenmodul (61, 62) an, das auf dem Mundstück angeordnet ist, um hin- und hergehendes Putzen durchzuführen, um so relevante Teile der Zähne zu reinigen;
Operationsmodus II:
die Anregungsquelle generiert alternierende Anregungssignale, um die Ausbuchtung so zu steuern, dass hin- und hergehende Begradigungs- und Biegeaktionen durchgeführt werden, um so relevante Teile der Zähne zu reinigen;
Operationsmodus III:
die Anregungsquelle sprüht Flüssigkeit, Gas oder aus Dampf und Flüssigkeit gemischten Nebel auf die relevante Position des Zahns durch die Rohrleitungen (31) und die kleinen Lochöffnungen (32) hindurch, um so die relevante Position des Zahns zu reinigen.

8. Zahnreiniger nach Anspruch 7, wobei die Reinigungsoperation im Operationsmodus I durchgeführt wird,
ein Anregungssignalsystem des dreidimensionalen Mundstückmodells gemäß einem Betätigungsmodulsystem des dreidimensionalen Mundstückmodells erstellt wird;
das Betätigungsmodulsystem des dreidimensionalen Mundstückmodells gemäß einem Borstenmodulsystem des dreidimensionalen Mundstückmodells erstellt wird;
das Borstenmodulsystem des dreidimensionalen Mundstückmodells gemäß dem Furchenoperationsbeschreibungssystem des dreidimensionalen Mundstückmodells erstellt wird;
wobei das Bezahnungspositionsbeschreibungssystem, das Furchenoperationsbeschreibungssystem, das Borstenmodulsystem, das Betätigungsmodulsystem und das Anregungssignalsystem sequentiell entsprechende Zuordnungsbeziehungen haben,
wobei vorzugsweise
das Anregungssignalsystem aus der Anregungssignalquelle und Anregungssignaldaten der Anregungssignalquelle besteht, wobei die Anregungssignalquelle innerhalb des Mundstückmodells angeordnet ist, um das Betätigungsmodul zu steuern, um mechanische Bewegung zu generieren, und die Anregungssignaldaten der Anregungssignalquelle gemäß den Betätigungsanforderungsdaten von jedem Betätigungsmodul erstellt werden,
das Betätigungsmodulsystem aus den Betätigungsmodulen und räumlichen Borstenlayoutdaten und den Putzanforderungsdaten von jedem der Betätigungsmodule besteht, wobei jedes der Betätigungsmodule in Phatnoma (Kassettenfeldern) des Mundstückmodells angeordnet ist und jedes Borstenmodul (61, 62) zur Aktion antreibt, und die räumlichen Betätigungslayoutdaten und die Betätigungsanforderungsdaten jedes Betätigungsmoduls gemäß den räumlichen Borstenlayoutdaten und den Putzanforderungsdaten jedes Borstenmoduls (61, 62) erstellt werden,
das Borstenmodulsystem aus den Borstenmodulen (61, 62) und räumlichen Furchenlayoutdaten und Operationsanforderungsdaten von jedem der Borstenmodule (61, 62) besteht, wobei jedes der Borstenmodule (61, 62) auf jeder Furchenoperationsbeschreibung angeordnet ist und die räumlichen Borstenlayoutdaten und die Putzanforderungsdaten jedes Borstenmoduls (61, 62) gemäß räumlichen Furchenlayoutdaten und Operationsanforderungsdaten jeder Furchenoperationsbeschreibung und räumlichen Bezahnungslayoutdaten jeder entsprechenden Bezahnungspositionsbeschreibung erstellt werden.

9. Zahnreiniger nach Anspruch 7, wobei die Reinigungsoperation im Operationsmodus II durchgeführt wird,
ein Rohrleitungssystem des dreidimensionalen Mundstückmodells gemäß einem Ausbuchtungsmodulsystem des dreidimensionalen Mundstückmodells erstellt wird; und
das Ausbuchtungsmodulsystem des dreidimensionalen Mundstückmodells gemäß dem Furchenoperationsbeschreibungssystem des dreidimensionalen Mundstückmodells erstellt wird;
wobei das Bezahnungspositionsbeschreibungssystem, das Furchenoperationsbeschreibungssystem, das Ausbuchtungsmodulsystem und das Rohrleitungssystem sequentiell entsprechende Zuordnungsbeziehungen haben;
wobei vorzugsweise
das Rohrleitungssystem aus den Rohrleitungen (31) und den räumlichen Rohrleitungslayoutdaten von jeder der Rohrleitungen (31) besteht, wobei die Rohrleitungen (31) in den Phatnoma des Mundstückmodells angeordnet sind, um ein Anregungssignal zu jeder Ausbuchtung auf jedem Ausbuchtungsmoduls zu transportieren, und umfassend eine Hauptrohrleitung und jede Zweitrohrleitung, und wobei die räumlichen Rohrleitungslayoutdaten jeder Rohrleitung (31) gemäß räumlichen Layoutdaten und Putzanforderungsdaten jedes Ausbuchtungsmoduls erstellt werden;
das Ausbuchtungsmodulsystem aus den Ausbuchtungsmodulen und räumlichen Ausbuchtungslayoutdaten und Putzanforderungsdaten von jedem der Ausbuchtungsmodule besteht, wobei die räumlichen Ausbuchtungslayoutdaten und die Putzanforderungsdaten von jedem Ausbuchtungsmodul gemäß räumlichen Layoutdaten und Operationsanforderungsdaten jeder Furchenoperationsbeschreibung und räumlichen Layoutdaten jeder entsprechenden Bezahnungspositionsbeschreibung erstellt werden; und
wobei das Anregungssignal ein alternierendes Luftdrucksignal, ein hydraulisches Drucksignal oder ein Spannungssignal ist.

10. Zahnreiniger nach Anspruch 7, wobei die Reinigungsoperation im Operationsmodus III durchgeführt wird,
ein Rohrleitungssystem des dreidimensionalen Mundstückmodells gemäß einem kleinen Lochöffnungssystem des dreidimensionalen Mundstückmodells erstellt wird; und
das kleine Lochöffnungssystem des dreidimensionalen Mundstückmodells gemäß dem Furchenoperationsbeschreibungssystem des dreidimensionalen Mundstückmodells erstellt wird;
wobei das Bezahnungspositionsbeschreibungssystem, das Furchenoperationsbeschreibungssystem, das kleine Lochöffnungssystem und das Rohrleitungssystem sequentiell entsprechende Zuordnungsbeziehungen haben;
wobei vorzugsweise
das Rohrleitungssystem aus Rohrleitungen (31) und den räumlichen Layoutdaten jeder Rohrleitung (31) besteht, wobei die Rohrleitungen (31) in den Phatnoma des Mundstückmodells angeordnet sind, um Anregungssignale zu jeder kleinen Lochöffnung (32) zu transportieren, und umfassend eine Hauptrohrleitung und jede Zweigrohrleitung, wobei die räumlichen Layoutdaten jeder Rohrleitung (31) gemäß den räumlichen Layoutdaten jeder kleinen Lochöffnung erstellt werden;
das kleine Lochöffnungssystem aus den kleinen Lochöffnungen (32) und räumlichen Layoutdaten jeder kleinen Lochöffnung (32) besteht, wobei die kleinen Lochöffnungen (32) auf jeder Furchenoperationsbeschreibung angeordnet sind, und räumliche Layoutdaten von jeder kleinen Lochöffnung (32) gemäß räumlichen Furchenlayoutdaten und Operationsanforderungsdaten jeder Furchenoperationsbeschreibung und räumlichen Bezahnungslayoutdaten von jeder entsprechenden Bezahnungspositionsbeschreibung erstellt werden;
wobei die Anregungsquelle Flüssigkeit, Gas oder aus Gas und Flüssigkeit gemischten Nebel auf eine relevante Position des Zahns durch die Rohrleitungen (31) und die kleinen Lochöffnungen (32) hindurch sprüht, um so die relevante Position des Zahns zu reinigen; und wobei das Anregungssignal ein alternierendes Luftdrucksignal oder ein hydraulisches Drucksignal ist.

11. Zahnreiniger nach einem der Ansprüche 7 bis 10, wobei jede Zahnfleischsulkuspositionsbeschreibung so festgelegt ist, dass sie mindestens zwei der Zahnfleischsulkusoperationsbeschreibungen entspricht, oder jede Zahnfleischsulkusoperationsbeschreibung so festgelegt ist, dass sie mindestens zwei der Borstenmodule (61, 62) entspricht, worauf sie angeordnet werden soll;
und/oder
für eine gegebene Zahnfleischsulkusposition jedes Borstenmodul (61, 62) so festgelegt wird, dass es gesteuert werden soll, um durch zwei der Betätigungsmodule zu putzen, die alternierend das Borstenmodul (61, 62) steuern, um alternierend in einer tangentialen Richtung einer Zahnfleischsulkuskurve und einer Normalrichtung der Zahnfleischsulkuskurve zu putzen.

12. Zahnreiniger nach einem der Ansprüche 7 bis 11, wobei das Anregungssignal ein alternierendes Luftdrucksignal, ein hydraulisches Drucksignal oder ein Spannungssignal ist.

13. Zahnreiniger nach einem der Ansprüche 7 bis 12, wobei das Betätigungsmodul aus einer Antriebseinheit und einer Übertragungseinheit zusammengesetzt ist, wobei die Antriebseinheit mit der Anregungsquelle verbunden ist und ein Anregungssignal empfängt, das von der Anregungsquelle generiert wurde, und die Übertragungseinheit zur Aktion antreibt, und wobei die Übertragungseinheit mit dem Borstenmodul (61, 62) verbunden ist, um so das Borstenmodul (61, 62) zur Aktion in einer vorab festgelegten Weise anzutreiben,
wobei die Antriebseinheit vorzugsweise eine pneumatische Antriebseinheit, eine hydraulische Antriebseinheit oder eine Spannungsantriebseinheit ist; und die Übertragungseinheit ein 7-förmiger Verbindungsstab oder ein T-förmiger Stützstab ist, wobei der 7-förmige Verbindungsstab einen ersten Kreuzstab und einen ersten vertikalen Griff umfasst, die integral gebildet sind, wobei der T-förmige Stützstab einen zweiten Kreuzstab und einen zweiten vertikalen Griff umfasst, die integral gebildet sind.

14. Zahnreiniger nach einem der Ansprüche 7 bis 12, wobei das Betätigungsmodul mindestens ein künstlicher Muskel (41, 71-72, 91-94) ist, das Borstenmodul (61, 62) aus einem Beflockungslöffel (61) und einer Borste (62) oder einem Cluster von Borsten (62), fixiert auf dem Beflockungslöffel (61), zusammengesetzt ist, ein Ende des künstlichen Muskels (41, 71-72, 91-94) mit dem Beflockungslöffel (61) verbunden ist, die Anregungsquelle ein Spannungssignal ist und die Anregungsquelle den künstlichen Muskel (41, 71-72, 91-94) steuert, um durch Anlegen eines Spannungssignals an den künstlichen Muskel (41, 71-72, 91-94) diesen zu kontrahieren und zu bewegen, um den Löffel zur Bewegung anzutreiben,
wobei vorzugsweise der mindestens eine künstliche Muskel (41, 71-72, 91-94) in mindestens zwei (71-72, 91-94) Exemplaren bereitgestellt wird, und bei der Zahnfleischsulkusposition ein Borstenmodul (61, 62) so festgelegt wird, dass es gesteuert wird, um durch die mindestens zwei künstlichen Muskeln (71-72, 91-94) zu putzen, wobei die künstlichen Muskeln (71-72, 91-94) um den Beflockungslöffel (61) des Borstenmoduls (61, 62) herum angeordnet sind, die künstlichen Muskeln (71-72, 91-94) so festgelegt sind, dass sie durch eine Vielzahl von Sätzen von Anregungssignalen mit Phasendifferenzen angetrieben werden, wobei die Vielzahl der Sätze von Anregungssignalen ermöglicht, dass ein Teil der künstlichen Muskeln (71-72, 91-94) durch Zusammenwirken von unterschiedlichen Spannungsphasendifferenzen kontrahiert, und das Borstenmodul (61, 62) angetrieben wird, um sich in Richtung einer Kontraktionsrichtung des künstlichen Muskels (71-72, 91-94) zu bewegen.

15. Zahnreiniger nach einem der Ansprüche 2 bis 14, wobei in der Korrelationsdetektierung die alternierenden Anregungssignale durch ein Anregungsmodul ausgelöst werden, welches detektiert, dass ein Druckwert, der durch Anliegen des Bissschlussdetektierungsbereichs einer Mundstückfurche und des Bissschlusspositionsbereichs einer Bezahnung des Benutzers generiert wird, den Schwellenwert erreicht und die Zeitdauer das vorgegebene Zeitintervall erreicht.

## Revendications

1. Nettoyeur de dents de type embout buccal, comprenant :
des conduits (31) encastrés dans l'embout buccal, une extrémité des conduits (31) étant pourvue d'ouvertures à petits trous (32) ;
le nettoyeur de dents comprenant en outre :
une source d'excitation, configurée pour générer un ou plusieurs groupes de signaux d'excitation alternés, une ou plusieurs opérations de nettoyage, avec différentes actions de nettoyage appliquées à diverses parties des dents, étant générées en fonction des signaux d'excitation ;
un module de poils (61, 62) agencé sur l'embout buccal ;
un renflement creux (21) communiquant avec un canal des conduits (31) et agencé sur une surface de paroi des conduits (31) ; et
une pluralité de modules d'actionnement reliés à la source d'excitation et agencés dans l'embout buccal ;
ladite une extrémité des conduits (31) correspondant à une position pertinente de la dent ;
une autre extrémité des conduits (31) étant reliée à la source d'excitation ; et
dans l'embout buccal, une corrélation entre l'opération de nettoyage et l'action de nettoyage et chaque partie de la dent appliquée étant établie à l'avance, la corrélation permettant de nettoyer individuellement chaque position de la dent de l'utilisateur.

2. Nettoyeur de dents selon la revendication 1,
la corrélation étant établie par le port de l'embout buccal pour effectuer une détection de corrélation dans le processus d'occlusion et déclencher la génération d'un signal d'excitation ;
l'embout buccal étant fabriqué selon un modèle d'embout buccal tridimensionnel numérique, et
le modèle d'embout buccal tridimensionnel numérique étant établi en fonction d'un modèle de dent tridimensionnel, et étant adapté au modèle de dent tridimensionnel, un système de nettoyage du modèle d'embout buccal tridimensionnel et chaque partie des dents du modèle de dent tridimensionnel ayant une disposition de relation spatiale correspondante ; et
le modèle de dent tridimensionnel étant établi en fonction d'une image d'agencement spatial d'une cavité buccale d'un utilisateur.

3. Nettoyeur de dents selon la revendication 2,
un système d'opération de nettoyage du système de nettoyage étant établi en fonction d'un système de traçage de rainures du modèle d'embout buccal tridimensionnel ;
le système de traçage de rainures étant établi en fonction d'un système de tracé de position de dentition du modèle de dent tridimensionnel ; et
le système de tracé de position de dentition étant établi en traçant une surface extérieure de la dentition du modèle de dent tridimensionnel en fonction du modèle de dent tridimensionnel d'une zone de positionnement d'occlusion sélectionnée,
des zones de détection de l'occlusion sur des parties correspondantes de la rainure supérieure et de la rainure inférieure du modèle d'embout buccal tridimensionnel étant marquées en fonction de la zone de positionnement d'occlusion, et
la zone de positionnement d'occlusion étant une ou plusieurs positions sélectionnées sur la dentition supérieure et la dentition inférieure du modèle de dent tridimensionnel.

4. Nettoyeur de dents selon la revendication 3, ledit système de tracé de position de dentition établi en traçant la surface extérieure de la dentition du modèle de dent tridimensionnel en fonction du modèle de dent tridimensionnel de la zone de positionnement d'occlusion sélectionnée comprenant :
le système de tracé de position de dentition du modèle de dent consiste en une pluralité de tracés de position de dentition mutuellement reliés, et en des données de disposition spatiale de dentition et des données d'exigences de nettoyage de chacun des tracés de position de dentition ;
la pluralité de tracés de position de dentition étant établie en traçant la zone de surface, à l'exception de la zone de positionnement d'occlusion, de la surface extérieure de la dentition du modèle en fonction du modèle de dent tridimensionnel dans la zone de positionnement d'occlusion sélectionnée, différents paramètres d'exigences de nettoyage initiaux étant prédéfinis pour des tracés de position de différentes classes à l'avance, et les paramètres d'exigences de nettoyage initiaux comprenant la sélection des modes d'opération de nettoyage ;
les données de disposition spatiale de dentition et les données d'exigences de nettoyage des tracés de position de dentition étant établies en fonction des informations d'image des tracés de position de dentition et des paramètres d'exigences de nettoyage initiaux des tracés de position de dentition.

5. Nettoyeur de dents selon la revendication 4, ledit système de traçage de rainures étant établi en fonction d'un système de tracé de position de dentition du modèle de dent tridimensionnel comprenant :
le système de traçage de rainures étant constitué d'une pluralité de traçages de rainures, et de données de disposition spatiale des rainures et de données d'exigences opérationnelles de chacun de la pluralité de traçages de rainures ;
chacune des données d'exigences opérationnelles étant établie en fonction des données de disposition spatiale de chacun des traçages de rainures, des données de disposition spatiale de chacun des tracés de position de dentition et des données d'exigences de nettoyage correspondant au mode d'opération de nettoyage sélectionné ; et
chacun de la pluralité de traçages de rainures étant établi en marquant les traçages de rainures sur des parties correspondantes de la zone de surface, à l'exception de la zone de détection de l'occlusion, de la surface de la rainure supérieure et de la rainure inférieure du modèle d'embout buccal tridimensionnel, en fonction de la relation correspondante avec la dentition supérieure et la dentition inférieure du modèle de dent, et en fonction des données de disposition spatiale de dentition de chaque tracé de position de dentition et des données d'exigences de nettoyage correspondant au mode d'opération de nettoyage sélectionné ;
un écart existant entre chaque traçage de rainures et chaque tracé de position de dentition correspondant lorsque la zone de détection d'occlusion est en butée contre la zone de positionnement d'occlusion au moyen des données de disposition spatiale des rainures.

6. Nettoyeur de dents selon la revendication 5,
en fonction des différentes exigences de nettoyage de chaque position de la dent, les tracés de position de dentition étant classés en catégories comprenant : le tracé de position du sillon gingival, le tracé de position de la surface de la dent, le tracé de position du diastème, le tracé de position de la surface occlusale de la dent, le tracé de position de la section de la dent et le tracé de position de la surface distale et médiane de la molaire distale ; et
selon le type de tracé du tracé de position de dentition, le traçage de rainures étant divisé en catégories correspondantes comprenant : le traçage du sillon gingival, le traçage de la surface de la dent, le traçage du diastème, le traçage de la surface occlusale de la dent, le traçage de la section de la dent et le traçage de la surface distale et médiane de la molaire distale.

7. Nettoyeur de dents selon l'une quelconque des revendications 2 à 6, l'opération de nettoyage étant générée par le système de nettoyage, et le mode d'opération de nettoyage étant basé sur une ou plusieurs combinaisons des trois modes suivants :
mode d'opération I :
les modules d'actionnement reçoivent le signal d'excitation et génèrent un ou plusieurs groupes de mouvements mécaniques ; et
le mouvement mécanique entraîne le module de poils (61, 62) agencé sur l'embout buccal pour effectuer un brossage en va-et-vient de manière à nettoyer les parties pertinentes des dents ;
mode d'opération II :
la source d'excitation génère des signaux d'excitation alternés pour commander le renflement afin qu'il effectue des actions de redressement et de flexion en va-et-vient de manière à nettoyer les parties pertinentes des dents ;
mode d'opération III :
la source d'excitation pulvérise un liquide, un gaz ou une nébulisation mixte vapeur-liquide sur la position pertinente de la dent à travers les conduits (31) et les ouvertures à petits trous (32) de manière à nettoyer la position pertinente de la dent.

8. Nettoyeur de dents selon la revendication 7, lorsque l'opération de nettoyage est effectuée en mode d'opération I,
un système de signal d'excitation du modèle d'embout buccal tridimensionnel étant établi en fonction d'un système de module d'actionnement du modèle d'embout buccal tridimensionnel ;
le système de module d'actionnement du modèle d'embout buccal tridimensionnel étant établi en fonction d'un système de modules de poils du modèle d'embout buccal tridimensionnel ;
le système de modules de poils du modèle d'embout buccal tridimensionnel étant établi en fonction du système de traçage de rainures du modèle d'embout buccal tridimensionnel ;
le système de tracé de position de dentition, le système de traçage de rainures, le système de modules de poils, le système de modules d'actionnement et le système de signaux d'excitation ayant des relations de mappage séquentielles correspondantes,
de préférence,
le système de signaux d'excitation comprenant la source de signaux d'excitation et les données de signaux d'excitation de la source de signaux d'excitation, la source de signaux d'excitation étant agencée à l'intérieur du modèle d'embout buccal pour commander le module d'actionnement afin de générer un mouvement mécanique, et les données de signaux d'excitation de la source de signaux d'excitation étant établies en fonction des données d'exigences d'actionnement de chaque module d'actionnement,
le système de modules d'actionnement comprenant les modules d'actionnement, et les données de disposition spatiale des poils et les données d'exigences de brossage de chacun des modules d'actionnement, chacun des modules d'actionnement étant agencé dans le phatnome du modèle d'embout buccal et entraînant chaque module de poils (61, 62) à agir, et les données de disposition spatiale d'actionnement et les données d'exigences d'actionnement de chaque module d'actionnement étant établies en fonction des données de disposition spatiale des poils et des données d'exigences de brossage de chaque module de poils (61, 62),
le système de modules de poils comprenant les modules de poils (61, 62), et des données de disposition spatiale des rainures et les données d'exigences opérationnelles de chacun des modules de poils (61, 62), chacun des modules de poils (61, 62) étant agencé sur chaque traçage de rainures, et les données de disposition spatiale des poils et les données d'exigences de brossage de chaque module de poils (61, 62) étant établies en fonction des données de disposition spatiale des rainures et des données d'exigences opérationnelles de chaque traçage de rainures et des données de disposition spatiale de dentition de chaque tracé de position de dentition correspondant.

9. Nettoyeur de dents selon la revendication 7, lorsque l'opération de nettoyage est effectuée en mode d'opération II,
un système de conduit du modèle d'embout buccal tridimensionnel étant établi en fonction d'un système de modules de renflement du modèle d'embout buccal tridimensionnel ; et
le système de modules de renflement du modèle d'embout buccal tridimensionnel étant établi en fonction du système de traçage de rainures du modèle d'embout buccal tridimensionnel ;
le système de tracé de position de dentition, le système de traçage de rainures, le système de modules de renflement et le système de conduits ayant des relations de mappage séquentielles correspondantes ;
de préférence,
le système de conduits comprenant les conduits (31) et les données de disposition spatiale de conduit de chacun des conduits (31), les conduits (31) étant agencés dans le phatnome du modèle d'embout buccal, pour transmettre un signal d'excitation à chaque renflement sur chaque module de renflement, et comprenant un conduit principal et chaque conduit ramifié, et les données de disposition spatiale de conduits de chaque conduit (31) étant établies en fonction des données de disposition spatiale et des données d'exigences de brossage de chaque module de renflement ;
le système de modules de renflement comprenant les modules de renflement, et des données de disposition spatiale de renflement et des données d'exigences de brossage de chacun des modules de renflement, les données de disposition spatiale de renflement et les données d'exigences de brossage de chaque module de renflement étant établies en fonction des données de disposition spatiale et des données d'exigences opérationnelles de chaque traçage de rainures et des données de disposition spatiale de chaque tracé de position de dentition correspondant ; et
le signal d'excitation étant un signal de pression d'air alterné, un signal de pression hydraulique ou un signal de tension.

10. Nettoyeur de dents selon la revendication 7, lorsque l'opération de nettoyage est effectuée en mode d'opération III,
un système de conduit du modèle d'embout buccal tridimensionnel étant établi en fonction d'un système d'ouvertures à petits trous du modèle d'embout buccal tridimensionnel ; et
le système d'ouvertures à petits trous du modèle d'embout buccal tridimensionnel étant établi en fonction du système de traçage de rainures du modèle d'embout buccal tridimensionnel ;
le système de tracé de position de dentition, le système de traçage de rainures, le système d'ouvertures à petits trous et le système de conduit ayant des relations de mappage séquentielles correspondantes ;
de préférence,
le système de conduits comprenant des conduits (31) et les données de disposition spatiale de chaque conduit (31), les conduits (31) étant agencés dans le phatnome du modèle d'embout buccal, pour transmettre des signaux d'excitation vers chaque ouverture à petit trou (32), et comprenant un conduit principal et chaque conduit ramifié, les données de disposition spatiale de chaque conduit (31) étant établies en fonction des données de disposition spatiale de chaque ouverture à petit trou ;
le système d'ouvertures à petits trous comprenant les ouvertures à petits trous (32) et des données de disposition spatiale de chaque ouverture à petit trou (32), les ouvertures à petits trous (32) étant agencées sur chaque traçage de rainures, et des données de disposition spatiale de chaque ouverture à petit trou (32) étant établies en fonction des données de disposition spatiale des rainures et des données d'exigences opérationnelles de chaque traçage de rainures et des données de disposition spatiale de dentition de chaque tracé de position de dentition correspondant ;
la source d'excitation pulvérisant un liquide, un gaz ou une nébulisation mixte gaz-liquide sur une position pertinente de la dent à travers les conduits (31) et les ouvertures à petits trous (32) de manière à nettoyer la position pertinente de la dent ; et
le signal d'excitation étant un signal de pression d'air alterné ou un signal de pression hydraulique.

11. Nettoyeur de dents selon l'une quelconque des revendications 7 à 10, chaque tracé de position du sillon gingival étant réglé pour correspondre à au moins deux des traçages du sillon gingival, ou chaque traçage du sillon gingival étant réglé pour correspondre à au moins deux des modules de poils (61, 62) à agencer sur le nettoyeur de dents ;
et/ou
pour une position du sillon gingival, chaque module de poils (61, 62) étant réglé pour être commandé pour brosser par deux des modules d'actionnement qui commandent alternativement le module de poils (61, 62) afin de brosser dans une direction tangentielle d'une courbe du sillon gingival et dans une direction normale de la courbe du sillon gingival alternativement.

12. Nettoyeur de dents selon l'une quelconque des revendications 7 à 11, le signal d'excitation étant un signal de pression d'air alterné, un signal de pression hydraulique ou un signal de tension.

13. Nettoyeur de dents selon l'une quelconque des revendications 7 à 12, le module d'actionnement étant composé d'une unité d'entraînement et d'une unité de transmission, l'unité d'entraînement étant reliée à la source d'excitation et recevant un signal d'excitation généré par la source d'excitation et entraînant l'unité de transmission à agir, et l'unité de transmission étant reliée au module de poils (61, 62) de manière à entraîner le module de poils (61, 62) à agir d'une manière préétablie,
de préférence, l'unité d'entraînement étant une unité d'entraînement pneumatique, une unité d'entraînement hydraulique ou une unité d'entraînement à tension ; et l'unité de transmission étant une tige de connexion en forme de 7 ou une tige de support en forme de T, la tige de connexion en forme de 7 comprenant une première tige transversale et une première poignée verticale formées d'un seul tenant, la tige de support en forme de T comprenant une seconde tige transversale et une seconde poignée verticale formées d'un seul tenant.

14. Nettoyeur de dents selon l'une quelconque des revendications 7 à 12, le module d'actionnement étant au moins un muscle artificiel (41, 71-72, 91-94), le module de poils (61, 62) étant composé d'un plateau de flocage (61) et d'un poil (62) ou d'un groupe de poils (62) fixé sur le plateau de flocage (61), une extrémité du muscle artificiel (41, 71-72, 91-94) étant reliée au plateau de flocage (61), la source d'excitation étant un signal de tension, et la source d'excitation commandant au muscle artificiel (41, 71-72, 91-94) de se contracter et de se déplacer en appliquant un signal de tension au muscle artificiel (41, 71-72, 91-94) de manière à entraîner le déplacement du plateau,
de préférence, l'au moins un muscle artificiel (41, 71-72, 91-94) étant fourni dans au moins deux (71-72, 91-94), et pour la position du sillon gingival, un module de poils (61, 62) étant réglé pour être commandé pour brosser par au moins deux muscles artificiels (71-72, 91-94), les muscles artificiels (71-72, 91-94) étant agencés autour du plateau de flocage (61) du module de poils (61, 62), les muscles artificiels (71-72, 91-94) étant réglés pour être commandés par une pluralité d'ensembles de signaux d'excitation avec des différences de phase, la pluralité d'ensembles de signaux d'excitation permettant à une partie des muscles artificiels (71-72, 91-94) de se contracter par la coopération de différentes différences de phase de tension, et le module de poils (61, 62) étant commandé pour se déplacer vers une direction de contraction du muscle artificiel (71-72, 91-94).

15. Nettoyeur de dents selon l'une quelconque des revendications 2 à 14, dans la détection de corrélation, l'alternance des signaux d'excitation étant déclenchée par un module d'excitation détectant qu'une valeur de pression générée par la mise en butée de la zone de détection d'occlusion d'une rainure d'embout buccal et de la zone de positionnement d'occlusion d'une dentition d'utilisateur atteint la valeur seuil et que la durée atteint l'intervalle de temps défini.
